(19) Europäisches Patentamt
European Patent Office
Office européen des brevets

(11) EP 3 941 958 B1

(12) **EUROPÄISCHE PATENTSCHRIFT**

(45) Veröffentlichungstag und Bekanntmachung des Hinweises auf die Patenterteilung:
**07.06.2023 Patentblatt 2023/23**

(21) Anmeldenummer: **20710977.8**

(22) Anmeldetag: **19.03.2020**

(51) Internationale Patentklassifikation (IPC):
***C08G 18/79*** *(2006.01)* ***C08G 18/32*** *(2006.01)*
***C08G 18/38*** *(2006.01)*

(52) Gemeinsame Patentklassifikation (CPC):
**C08G 18/3821; C08G 18/3234; C08G 18/325; C08G 18/797**

(86) Internationale Anmeldenummer:
**PCT/EP2020/057586**

(87) Internationale Veröffentlichungsnummer:
**WO 2020/188024 (24.09.2020 Gazette 2020/39)**

(54) **SEKUNDÄRE AMINOGRUPPEN HALTIGE BINDEMITTEL BASIEREND AUF CYCLISCHEN ETHERN**

SECONDARY AMINO GROUP CONTAINING BINDERS BASED ON CYCLIC ETHERS

GROUPES AMINÉS SECONDAIRES CONTENANT UN LIANT À BASE D'ÉTHERS CYCLIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **19.03.2019 EP 19163663**

(43) Veröffentlichungstag der Anmeldung:
**26.01.2022 Patentblatt 2022/04**

(73) Patentinhaber: **Covestro Intellectual Property GmbH & Co. KG
51373 Leverkusen (DE)**

(72) Erfinder:
- **LATORRE MARTINEZ, Irene, Cristina
51373 Leverkusen (DE)**
- **GRESZTA-FRANZ, Dorota
42659 Solingen (DE)**
- **PIRES, Raul
50670 Köln (DE)**
- **YUVA, Nusret
51399 Burscheid (DE)**
- **FLÜGEL, Magdalena
51145 Köln (DE)**
- **HECKING, Andreas
40764 Langenfeld (DE)**
- **GOLLING, Florian
40237 Düsseldorf (DE)**

(74) Vertreter: **Levpat
c/o Covestro AG
Gebäude K12
51365 Leverkusen (DE)**

(56) Entgegenhaltungen:
**EP-A1- 3 098 247**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Processed by Luminess, 75001 PARIS (FR)

**Beschreibung**

Die vorliegende Erfindung betrifft ein Verfahren zur Herstellung von Polyasparaginsäureester-Zusammensetzungen durch Umsetzung von cyclischen, primäre Amino- und/oder primäre Aminoalkylgruppen tragenden Ethern mit Fumarsäure- und/oder Maleinsäureestern, die so erhältlichen Polyasparaginsäureester-Zusammensetzungen, sowie deren Verwendung in Zweikomponenten-Beschichtungsmitteln.

Zweikomponenten (2K)-Beschichtungsmittel, die als Bindemittel eine Polyisocyanatkomponente in Kombination mit einer gegenüber Isocyanatgruppen reaktionsfähigen Reaktivkomponente, insbesondere einer Polyhydroxylkomponente enthalten, sind seit langem bekannt. Sie eignen sich zur Herstellung von hochwertigen Überzügen, die hart, elastisch, abriebbeständig und vor allem auch witterungsstabil eingestellt werden können.

Innerhalb dieser 2K-Polyurethan-Beschichtungstechnologie haben sich in letzter Zeit bestimmte, estergruppenhaltige, sekundäre Polyamine etabliert, die sogenannten Polyasparaginsäureester oder Polyaspartate, die sich in Kombination mit Lackpolyisocyanaten insbesondere als Bindemittel in lösemittelarmen oder -freien (high solids) Beschichtungsmitteln eignen und eine rasche Aushärtung der Beschichtungen bei niedrigen Temperaturen ermöglichen.

Die Verwendung von Polyasparaginsäureestern allein oder in einem Gemisch mit weiteren, gegenüber Isocyanatgruppen reaktionsfähigen Komponenten in 2K-Beschichtungsmitteln wird beispielsweise in der EP0403921, EP0639628, EP0667362, EP0689881, US5214086, EP0699696, EP0596360, EP0893458, DE19701835, EP0470461, WO15130501, WO15130502 und US5243012 beschrieben.

Die Herstellung von aminofunktionellen Asparaginsäureestern ist an sich bekannt. Die Synthese erfolgt über eine Addition von primären Polyaminen an eine aktivierte Kohlenstoff-Doppelbindung vinyloger Carbonylverbindungen, wie beispielsweise in Malein- oder Fumarsäureestern enthalten, die in der Literatur hinreichend beschrieben ist (Houben-Weyl, Meth. d. Org. Chemie Bd. 11/1, 272 (1957), Usp. Khim. 1969, 38, 1933). In den kommerziell erhältlichen Polyasparaginsäureestern wird Maleinsäureester als die vinyloge Carbonylverbindung eingesetzt.

Die Aufgabe der vorliegenden Erfindung bestand in der Bereitstellung von Beschichtungszusammensetzungen auf Polyasparaginsäureesterbasis, die zu Beschichtungen führen mit -im Vergleich zu Beschichtungen auf Basis der aus dem Stand der Technik bekannten Polyasparaginsäureester-haltigen Beschichtungszusammensetzungen- deutlich verbesserter Lösemittelstabilität.

Überraschend wurde gefunden, dass diese Aufgabe durch die Verwendung von Polyasparaginsäureester-Zusammensetzungen, welche erhältlich sind durch Umsetzung von cyclischen, primäre Amino- und/oder primäre Aminoalkylgruppen tragenden Ethern mit Fumarsäure- und/oder Maleinsäureestern, gelöst wird.

EP 141 062 beschreibt die Umsetzung von primäre Aminogruppen tragenden Oxetanen und Tetrahydrofuranen mit Isocyanaten zu Prepolymeren, die weiter zu Elastomeren verarbeitet werden.

Polyasparaginsäureester-Zusammensetzungen, basierend auf cyclischen, primäre Amino- und/oder primäre Aminoalkylgruppen tragenden Ethern, und deren Verwendung in Beschichtungszusammensetzungen zur Herstellung lösemittelstabiler Beschichtungen sind aus dem Stand der Technik nicht bekannt.

Gegenstand der vorliegenden Erfindung sind Zusammensetzungen A1 enthaltend oder bestehend aus einen/einem oder mehrere/n Polyasparaginsäureester/n der allgemeinen Formel (I)

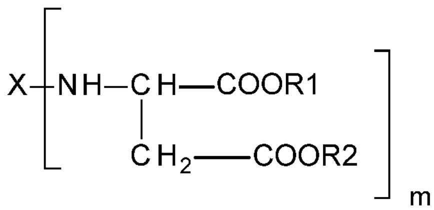

(I),

in welcher

| | |
|---|---|
| X | für einen m-wertigen organischen Rest steht, der erhalten werden kann durch Entfernung der primären Aminogruppen aus entsprechenden cyclischen Ethern, bei denen es sich um Monocyclen oder um anellierte Bicyclen auf Basis von monocyclischen Ethern handelt und die an wenigstens 2 der Ringkohlenstoffatome eine Gruppe tragen, die ausgewählt ist aus primärer Aminogruppe und aliphatisch gebundener primärer Aminogruppe, wobei |
| R1 und R2 | für gleiche oder verschiedene organische Reste, mit je 1 bis 18 Kohlenstoffatomen stehen, und |
| m | für eine ganze Zahl > 1 steht, |

und
gegebenenfalls einen/einem oder mehrere/n Polyasparaginsäureester/n mit primärer Aminogruppe der allgemeinen Formel (II)

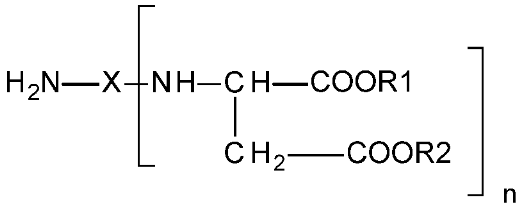

(II)

in welcher

n für m-1 steht und
X und die Reste R1 und R2 die oben genannten Bedeutungen haben.

Bevorzugt stehen R1 und R2 in Formel I und II für gleiche oder verschiedene Alkylreste mit je 1 bis 18 Kohlenstoffatomen, besonders bevorzugt gleiche oder verschiedene Alkylreste mit je 1 bis 8 Kohlenstoffatomen und ganz besonders bevorzugt für jeweils Alkylreste wie Methyl-, Ethyl-, Propyl-, iso-Propyl-, Butyl- oder iso-Butyl-Reste stehen. Am meisten bevorzugt ist Ethyl.

Bei den cyclischen Ethern, die an wenigstens 2 der Ringkohlenstoffatome eine Gruppe tragen, die ausgewählt ist aus primärer Aminogruppe und aliphatisch gebundener primärer Aminogruppe, und aus denen sich X ableitet, handelt es sich um Monocyclen oder Bicyclen gemäß der folgenden allgemeinen Formeln III und IV :

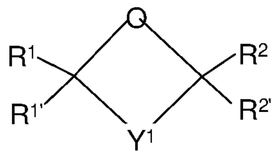

Formel III,

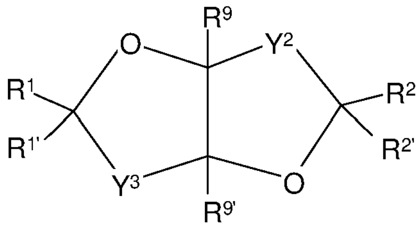

Formel IV,

wobei $Y^1$ für

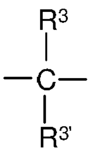 $Y^1a$ , 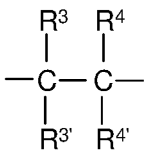 $Y^1b$ , 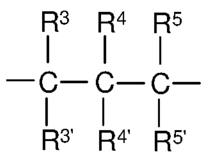 $Y^1c$ ,

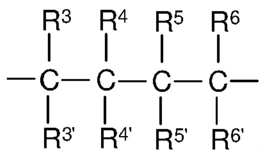 $Y^1d$ , 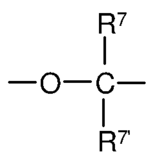 $Y^1e$ , 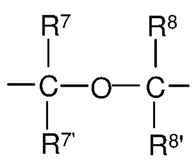 $Y^1f$ ,

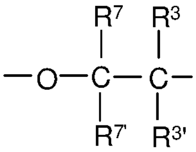 Y¹g , 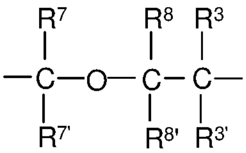 Y¹h , 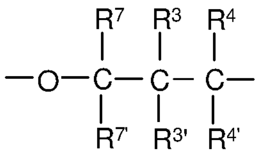 Y¹i

und $Y^2$ für

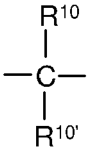 $Y^2a$ , 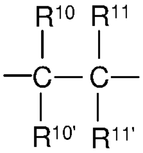 $Y^2b$ , 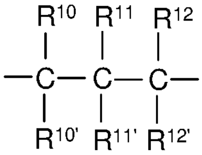 $Y^2c$ ,

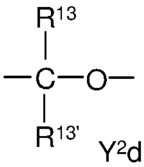 $Y^2d$ , 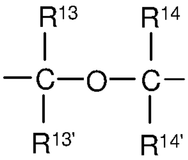 $Y^2e$ , 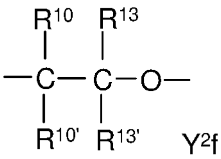 $Y^2f$

und $Y^3$ für

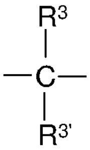 $Y^3a$ , 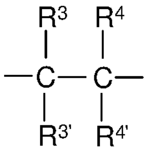 $Y^3b$ , 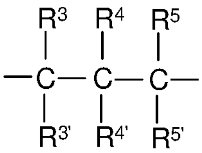 $Y^3c$ ,

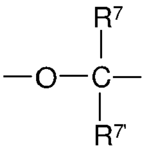 $Y^3d$ , 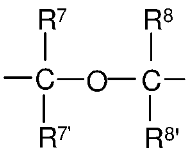 $Y^3e$ , 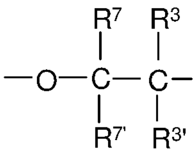 $Y^3f$

stehen, und

$R^1$, $R^2$, $R^7$, $R^8$, $R^{13}$, $R^{14}$ unabhängig voneinander für eine Alkylengruppe mit 1 bis 6 Kohlenstoffatomen oder Wasserstoff oder organische Reste stehen, wobei es sich bei letzteren um gesättigte oder ungesättigte, lineare oder verzweigte, aliphatische oder cycloaliphatische oder gegebenenfalls substituierte aromatische oder araliphatische einbindige Reste mit bis zu 18 Kohlenstoffatomen, die gegebenenfalls Heteroatome aus der Reihe Sauerstoff, Schwefel, Stickstoff enthalten können, handelt, wobei zu den Heteroatom-enthaltenden Resten z.B. solche gehören, die gegenüber Isocyanatgruppen reaktive und/oder bei Temperaturen bis 100°C inerte funktionelle Gruppen enthalten (ausgenommen primäre Aminogruppen),
und
$R^3$, $R^4$, $R^5$, $R^6$, $R^{10}$, $R^{11}$, $R^{12}$ unabhängig voneinander für eine Alkylengruppe mit 1 bis 6 Kohlenstoffatomen oder eine Bindung oder Wasserstoff oder organische Reste stehen, wobei es sich bei letzteren um gesättigte oder ungesättigte, lineare oder verzweigte, aliphatische oder cycloaliphatische oder gegebenenfalls substituierte aromatische oder araliphatische einbindige Reste mit bis zu 18 Kohlenstoffatomen, die gegebenenfalls Heteroatome aus der Reihe Sauerstoff, Schwefel, Stickstoff enthalten können, handelt, wobei zu den Heteroatom-enthaltenden Resten z.B. solche gehören, die gegenüber Isocyanatgruppen reaktive und/oder bei Temperaturen bis 100°C inerte funktionelle Gruppen enthalten (ausgenommen primäre Aminogruppen),
wobei
im Fall der Formel III mindestens 2 der Reste $R^1$ bis $R^8$ an eine $NH_2$-Gruppe gebunden sind (d.h. C1- bis C6-

Alkylengruppe-$NH_2$ (z.B. $CH_2$-$NH_2$) oder Bindung-$NH_2$), und
im Fall der Formel IV mindestens einer der Reste $R^1$ bis $R^8$ an eine $NH_2$-Gruppe gebunden ist und mindestens einer der Reste $R^{10}$ bis $R^{14}$ an eine $NH_2$-Gruppe gebunden ist (d.h. C1- bis C6-Alkylengruppe-$NH_2$ (z.B. $CH_2$-$NH_2$) oder Bindung-$NH_2$), und
$R^9$ und $R^{9'}$ unabhängig voneinander für H oder einen Methylrest stehen, und
$R^{1'}$ bis $R^{8'}$ und $R^{10'}$ bis $R^{14'}$ unabhängig voneinander für Wasserstoff oder organische Reste stehen, wobei es sich bei letzteren um gesättigte oder ungesättigte, lineare oder verzweigte, aliphatische oder cycloaliphatische oder gegebenenfalls substituierte aromatische oder araliphatische einbindige Reste mit bis zu 18 Kohlenstoffatomen, die gegebenenfalls Heteroatome aus der Reihe Sauerstoff, Schwefel, Stickstoff enthalten können, handelt, wobei zu den Heteroatom-enthaltenden Resten z.B. solche gehören, die gegenüber Isocyanatgruppen reaktive und/oder bei Temperaturen bis 100°C inerte funktionelle Gruppen enthalten (ausgenommen primäre Aminogruppen).

Die Alkylengruppen mit 1 bis 6 Kohlenstoffatomen können linear oder verzweigt sein, bevorzugt handelt es sich bei diesen Alkylengruppen um $CH_2$, $CH_2$-$CH_2$, $CH_2$-$CH_2$-$CH_2$ oder $CH_2$-$CH_2$-$CH_2$-$CH_2$, besonders bevorzugt um $CH_2$.

Falls die Reste $R^1$, $R^2$, $R^3$, $R^4$, $R^5$, $R^6$, $R^7$, $R^8$, $R^{10}$, $R^{11}$, $R^{12}$ $R^{13}$, $R^{14}$ für Wasserstoff oder organische Reste, so stehen sie bevorzugt für Wasserstoff und/oder Alkylreste mit je 1 bis 8 Kohlenstoffatomen und ganz besonders bevorzugt für Wasserstoff und/oder Alkylreste wie Methyl- , Ethyl-, Propyl-, iso-Propyl-, Butyl- oder iso-Butyl-Reste, am meisten bevorzugt für Wasserstoff.

Bevorzugt handelt es sich bei den Resten $R^{1'}$ bis $R^{8'}$ und $R^{10'}$ bis $R^{14}$ um Wasserstoff und/oder Alkylreste mit je 1 bis 8 Kohlenstoffatomen und ganz besonders bevorzugt um Wasserstoff und/oder Alkylreste wie Methyl-, Ethyl-, Propyl-, iso-Propyl-, Butyl- oder iso-Butyl-Reste, am meisten bevorzugt um Wasserstoff.

Die cyclischen Ether tragen an wenigstens 2 der Ringkohlenstoffatome eine Gruppe, die ausgewählt ist aus primärer Aminogruppe und aliphatisch gebundener primärer Aminogruppe.

Die cyclischen Ether der Formel III tragen bevorzugt an genau 3 oder 2 Ringkohlenstoffatomen, besonders bevorzugt an genau 2 Ringkohlenstoffatomen eine Gruppe, die ausgewählt ist aus primärer Aminogruppe und aliphatisch gebundener primärer Aminogruppe.

Die bicyclischen Ether der Formel III tragen bevorzugt an genau 2 Ringkohlenstoffatomen des einen Cyclus und an genau einem Ringkohlenstoffatom des zweiten Cyclus eine Gruppe, die ausgewählt ist aus primärer Aminogruppe und aliphatisch gebundener primärer Aminogruppe. Besonders bevorzugt trägt jeder der beiden Ringe an genau einem Ringkohlenstoffatom eine Gruppe, die ausgewählt ist aus primärer Aminogruppe und aliphatisch gebundener primärer Aminogruppe.

In den Fällen, in denen genau 3 Ringkohlenstoffatome eine Gruppe tragen, die ausgewählt ist aus primärer Aminogruppe und aliphatisch gebundener primärer Aminogruppe, ist in den Formeln I und II m=3. In den Fällen, in denen genau 2 Ringkohlenstoffatome eine solche Gruppe tragen, ist in den Formeln I und II m=2.

Bevorzugte Ausführungsformen der Formel III:
Bevorzugt stehen in Formel III $Y^1$ für

i) $Y^1$a, $R^1$ und $R^2$ für $CH_2$,
und $R^{1'}$, $R^{2'}$, $R^3$ und $R^{3'}$ unabhängig voneinander für organische Reste mit bis zu 18 Kohlenstoffatomen gemäß obiger Beschreibung oder für H, bevorzugt für Alkylreste mit 1 bis 8 Kohlenstoffatomen oder H,

ii) Y'b, 2 der Reste $R^1$ bis $R^4$ für $CH_2$,
und die beiden verbleibenden dieser Reste, sowie $R^{1'}$ bis $R^{4'}$ unabhängig voneinander für organische Reste mit bis zu 18 Kohlenstoffatomen gemäß obiger Beschreibung oder für H, bevorzugt für Alkylreste mit 1 bis 8 Kohlenstoffatomen oder H,
oder $R^3$ und $R^4$ für eine Bindung,
und $R^1$, $R^{1'}$, $R^2$, $R^{2'}$, $R^{3'}$ und $R^{4'}$unabhängig voneinander für organische Reste mit bis zu 18 Kohlenstoffatomen gemäß obiger Beschreibung oder für H, bevorzugt für Alkylreste mit 1 bis 8 Kohlenstoffatomen oder H,

iii) $Y^1$c, 2 der Reste $R^1$, $R^2$, $R^3$, $R^5$ für $CH_2$,
und die beiden verbleibenden dieser Reste, sowie $R^{1'}$ bis $R^5$ und $R^4$ unabhängig voneinander für organische Reste mit bis zu 18 Kohlenstoffatomen gemäß obiger Beschreibung oder für H, bevorzugt für Alkylreste mit 1 bis 8 Kohlenstoffatomen oder H,
oder $R^3$ und $R^4$ für eine Bindung,
und $R^1$, $R^2$, $R^5$, sowie $R^{1'}$ bis $R^{5'}$unabhängig voneinander für organische Reste mit bis zu 18 Kohlenstoffatomen gemäß obiger Beschreibung oder für H, bevorzugt für Alkylreste mit 1 bis 8 Kohlenstoffatomen oder H,

(fortgesetzt)

oder $R^3$ und $R^5$ für eine Bindung,
und $R^1$, $R^2$, $R^4$, sowie $R^{1'}$ bis $R^{5'}$ unabhängig voneinander für organische Reste mit bis zu 18 Kohlenstoffatomen gemäß obiger Beschreibung oder für H, bevorzugt für Alkylreste mit 1 bis 8 Kohlenstoffatomen oder H,
oder $R^1$ für $CH_2$ und $R^3$, $R^4$ oder $R^5$ für eine Bindung,
und die beiden verbleibenden Reste aus $R^3$, $R^4$ und $R^5$, sowie $R^{1'}$ bis $R^{5'}$ und $R^2$ unabhängig voneinander für organische Reste mit bis zu 18 Kohlenstoffatomen gemäß obiger Beschreibung oder für H, bevorzugt für Alkylreste mit 1 bis 8 Kohlenstoffatomen oder H,

iv) $Y^1$d, 2 der Reste $R^1$, $R^2$, $R^3$, $R^6$ für $CH_2$,
und die beiden verbleibenden dieser Reste, sowie $R^4$, $R^5$ und $R^{1'}$ bis $R^{6'}$ unabhängig voneinander für organische Reste mit bis zu 18 Kohlenstoffatomen gemäß obiger Beschreibung oder für H, bevorzugt für Alkylreste mit 1 bis 8 Kohlenstoffatomen oder H,
oder $R^3$ und $R^6$ für eine Bindung,
und $R^1$, $R^2$ $R^4$, $R^5$ und $R^{1'}$ bis $R^{6'}$ unabhängig voneinander für organische Reste mit bis zu 18 Kohlenstoffatomen gemäß obiger Beschreibung oder für H, bevorzugt für Alkylreste mit 1 bis 8 Kohlenstoffatomen oder H,
oder $R^1$ für $CH_2$ und $R^3$ oder $R^6$ für eine Bindung
und der verbleibende Rest aus $R^3$ und $R^6$, sowie $R^{1'}$ bis $R^{5'}$ und $R^2$ unabhängig voneinander für organische Reste mit bis zu 18 Kohlenstoffatomen gemäß obiger Beschreibung oder für H, bevorzugt für Alkylreste mit 1 bis 8 Kohlenstoffatomen oder H,

v) Y'e, 2 der Reste $R^1$, $R^2$, $R^7$ für $CH_2$,
und der verbleibende dieser 3 Reste, sowie $R^{1'}$, $R^{2'}$ und $R^{7'}$ unabhängig voneinander für organische Reste mit bis zu 18 Kohlenstoffatomen gemäß obiger Beschreibung oder für H, bevorzugt für Alkylreste mit 1 bis 8 Kohlenstoffatomen oder H,

vi) $Y^1$f, 2 der Reste $R^1$, $R^2$, $R^7$, $R^8$ für $CH_2$,
und die beiden verbleibenden dieser Reste, sowie $R^{1'}$, $R^{2'}$, $R^{7'}$ und $R^{8'}$ unabhängig voneinander für organische Reste mit bis zu 18 Kohlenstoffatomen gemäß obiger Beschreibung oder für H, bevorzugt für Alkylreste mit 1 bis 8 Kohlenstoffatomen oder H,

vii) Y'g, 2 der Reste $R^1$, $R^2$, $R^3$, $R^7$ für $CH_2$,
und die beiden verbleibenden dieser Reste, sowie $R^{1'}$, $R^{2'}$, $R^{3'}$ und $R^{7'}$ unabhängig voneinander für organische Reste mit bis zu 18 Kohlenstoffatomen gemäß obiger Beschreibung oder für H, bevorzugt für Alkylreste mit 1 bis 8 Kohlenstoffatomen oder H,
oder $R^1$ für $CH_2$ und $R^3$ für eine Bindung,
und $R^2$, $R^7$, $R^{1'}$, $R^{2'}$, $R^{3'}$ und $R^{7'}$ unabhängig voneinander für organische Reste mit bis zu 18 Kohlenstoffatomen gemäß obiger Beschreibung oder für H, bevorzugt für Alkylreste mit 1 bis 8 Kohlenstoffatomen oder H,

viii) Y'h, 2 der Reste $R^1$, $R^2$, $R^3$, $R^7$, $R^8$ für $CH_2$,
und die 3 verbleibenden dieser Reste, sowie $R^{1'}$, $R^{2'}$, $R^{3'}$, $R^{7'}$ und $R^{8'}$ unabhängig voneinander für organische Reste mit bis zu 18 Kohlenstoffatomen gemäß obiger Beschreibung oder für H, bevorzugt für Alkylreste mit 1 bis 8 Kohlenstoffatomen oder H,
oder $R^1$ für $CH_2$ und $R^3$ für eine Bindung,
und $R^2$, $R^7$, $R^8$ sowie $R^{1'}$, $R^{2'}$, $R^{3'}$, $R^{7'}$ und $R^{8'}$ unabhängig voneinander für organische Reste mit bis zu 18 Kohlenstoffatomen gemäß obiger Beschreibung oder für H, bevorzugt für Alkylreste mit 1 bis 8 Kohlenstoffatomen oder H,

ix) $Y^1$i, 2 der Reste $R^1$, $R^2$, $R^3$, $R^4$, $R^7$ für $CH_2$,
und die 3 verbleibenden dieser Reste, sowie $R^{1'}$, $R^{2'}$, $R^{3'}$, $R^{4'}$ und $R^{7'}$ unabhängig voneinander für organische Reste mit bis zu 18 Kohlenstoffatomen gemäß obiger Beschreibung oder für H, bevorzugt für Alkylreste mit 1 bis 8 Kohlenstoffatomen oder H,
oder $R^1$ für $CH_2$ und $R^3$ oder $R^4$ für eine Bindung,
und der verbleibende Rest aus $R^3$ und $R^4$, sowie $R^2$, $R^7$, $R^{1'}$, $R^{2'}$, $R^{3'}$, $R^{4'}$ und $R^{7'}$ unabhängig voneinander für organische Reste mit bis zu 18 Kohlenstoffatomen gemäß obiger Beschreibung oder für H, bevorzugt für Alkylreste mit 1 bis 8 Kohlenstoffatomen oder H,

Besonders bevorzugte Ausführungsformen der Formel III sind die Ausführungsformen ii), iii), v), vi) und vii).

Ganz besonders bevorzugt sind die Ausführungsformen ii) und iii), worin

ii) Y1 für Y'b, $R^1$ und $R^2$ für $CH_2$,

und $R^3$, $R^4$, sowie $R^{1'}$ bis $R^{4'}$ unabhängig voneinander für organische Reste mit bis zu 18 Kohlenstoffatomen gemäß obiger Beschreibung oder für H, bevorzugt für Alkylreste mit 1 bis 8 Kohlenstoffatomen oder H,

oder $R^3$ und $R^4$ für $CH_2$ oder eine Bindung,

und $R^1$, $R^2$,sowie $R^{1'}$ bis $R^{4'}$ unabhängig voneinander für organische Reste mit bis zu 18 Kohlenstoffatomen gemäß obiger Beschreibung oder für H, bevorzugt für Alkylreste mit 1 bis 8 Kohlenstoffatomen oder H, und

iii) Y1 für Y'c, $R^1$ und $R^2$ für $CH_2$,

und $R^3$, $R^4$, $R^5$, sowie $R^{1'}$ bis $R^{5'}$ unabhängig voneinander für organische Reste mit bis zu 18 Kohlenstoffatomen gemäß obiger Beschreibung oder für H, bevorzugt für Alkylreste mit 1 bis 8 Kohlenstoffatomen oder H,

oder $R^3$ und $R^5$ für CH2 oder eine Bindung,

und $R^1$, $R^2$, $R^4$, sowie $R^{1'}$ bis $R^{5'}$ unabhängig voneinander für organische Reste mit bis zu 18 Kohlenstoffatomen gemäß obiger Beschreibung oder für H, bevorzugt für Alkylreste mit 1 bis 8 Kohlenstoffatomen oder H, stehen.

Am meisten bevorzugt sind die Ausführungsformen ii) und iii), worin

ii) Y1 für Y'b, $R^1$ und $R^2$ für $CH_2$,
und $R^3$, $R^4$, sowie $R^{1'}$ bis $R^{4'}$ unabhängig voneinander für einen Methylrest oder H, bevorzugt für H,
oder $R^3$ und $R^4$ für $CH_2$ oder eine Bindung,
und $R^1$, $R^2$,sowie $R^{1'}$ bis $R^{4'}$ unabhängig voneinander für einen Methylrest oder H, bevorzugt für H, und

iii) Y1 für Y'c, $R^1$ und $R^2$ für $CH_2$,
und $R^3$, $R^4$, $R^5$, sowie $R^{1'}$ bis $R^{5'}$ unabhängig voneinander für einen Methylrest oder H, bevorzugt für H,
oder $R^3$ und $R^5$ für CH2 oder eine Bindung,
und $R^1$, $R^2$, $R^4$, sowie $R^{1'}$ bis $R^{5'}$ unabhängig voneinander für einen Methylrest oder H, bevorzugt für H

stehen.

Beispiele für die am meisten bevorzugten Verbindungen der Formel III sind Oxacyclopentan-2,3-, - 2,4-, -2,5- oder -3,4-di-methylenamin oder Oxacyclohexan-2,3-, -2,4-, -2,5-, -2,6-, -3,4- oder -3,5-di-methylenamin.

Bevorzugte Ausführungsformen der Formel IV:
Bevorzugt stehen in Formel IV $Y^2$ und $Y^3$ für

a) $Y^2$a und $Y^3$a, $R^2$für $CH_2$ oder $R^{10}$ für $CH_2$ oder eine Bindung und $R^1$ für $CH_2$ oder $R^3$ für $CH_2$ oder eine Bindung ,

und der verbleibende Rest aus $R^2$ und $R^{10}$, der verbleibende Rest aus $R^1$ und $R^3$, sowie $R^{1'}$, $R^{2'}$, $R^{3'}$ und $R^{10'}$ unabhängig voneinander für Methyl-, Ethyl-, Propyl-, iso-Propyl-, Butyl-, iso-Butyl-Reste oder H, bevorzugt für H, und $R^9$, $R^{9'}$ unabhängig voneinander für $CH_3$ oder H, bevorzugt für H,

b) $Y^2$a und $Y^3$b, $R^2$für $CH_2$ oder $R^{10}$ für $CH_2$ oder eine Bindung und $R^1$ für $CH_2$ oder $R^3$ oder $R^4$ für $CH_2$ oder eine Bindung,

und der verbleibende Rest aus $R^2$ und $R^{10}$, die verbleibenden Reste aus $R^1$, $R^3$ und $R^4$, sowie $R^{1'}$, $R^{2'}$, $R^{3'}$, $R^{4'}$ und $R^{10'}$ unabhängig voneinander für Methyl-, Ethyl-, Propyl-, iso-Propyl-, Butyl-, iso-Butyl-Reste oder H, bevorzugt für H, und $R^9$, $R^{9'}$ unabhängig voneinander für $CH_3$ oder H, bevorzugt für H,

c) $Y^2$a und $Y^3$d, $R^2$für $CH_2$ oder $R^{10}$ für $CH_2$ oder eine Bindung und $R^1$ oder $R^7$ für $CH_2$,

und der verbleibende Rest aus $R^2$ und $R^{10}$, der verbleibende Rest aus $R^1$ und $R^7$, sowie $R^{1'}$, $R^{2'}$, $R^{7'}$ und $R^{10'}$ unabhängig voneinander für Methyl-, Ethyl-, Propyl-, iso-Propyl-, Butyl-, iso-Butyl-Reste oder H, bevorzugt für H, und $R^9$, $R^{9'}$ unabhängig voneinander für $CH_3$ oder H, bevorzugt für H,

d) $Y^2$b und $Y^3$b, $R^2$für $CH_2$ oder $R^{10}$ oder $R^{11}$ für $CH_2$ oder eine Bindung und $R^1$ für $CH_2$ oder $R^3$ oder $R^4$ für $CH_2$ oder eine Bindung,

und die verbleibenden Reste aus $R^2$, $R^{10}$ und $R^{11}$, die verbleibenden Reste aus $R^1$, $R^3$ und $R^7$, sowie $R^{1'}$, $R^{2'}$, $R^{3'}$, $R^4$, $R^{10'}$ und $R^{11'}$ unabhängig voneinander für Methyl-, Ethyl-, Propyl-, iso-Propyl-, Butyl-, iso-Butyl-Reste oder H, bevorzugt für H, und $R^9$, $R^{9'}$ unabhängig voneinander für $CH_3$ oder H, bevorzugt für H,

e) $Y^2$d und $Y^3$d, $R^2$ oder $R^{13}$ für $CH_2$ und $R^1$ oder $R^7$ für $CH_2$,

und der verbleibende Rest aus $R^2$ und $R^{13}$, der verbleibende Rest aus $R^1$ und $R^7$, sowie $R^{1'}$, $R^{2'}$, $R^{7'}$ und $R^{13'}$ unabhängig voneinander für Methyl-, Ethyl-, Propyl-, iso-Propyl-, Butyl-, iso-Butyl-Reste oder H, bevorzugt für H, und $R^9$, $R^{9'}$ unabhängig voneinander für $CH_3$ oder H, bevorzugt für H,

f) $Y^2$b und $Y^3$d, $R^2$für $CH_2$ oder $R^{10}$ oder $R^{11}$ für $CH_2$ oder eine Bindung und $R^1$ oder $R^7$ für $CH_2$,

und die verbleibenden Reste aus $R^2$, $R^{10}$ und $R^{11}$, der verbleibende Rest aus $R^1$ und $R^7$, sowie $R^{1'}$, $R^{2'}$, $R^{7'}$, $R^{10'}$ und $R^{11'}$ unabhängig voneinander für Methyl-, Ethyl-, Propyl-, iso-Propyl-, Butyl-, iso-Butyl-Reste oder H, bevorzugt für H, und $R^9$, $R^{9'}$ unabhängig voneinander für $CH_3$ oder H, bevorzugt für H, stehen.

Besonders bevorzugte Ausführungsformen der Formel IV sind die Ausführungsformen a), b) und d). Ganz besonders bevorzugt sind die Ausführungsformen a), b) und d) mit der Einschränkung, dass $R^9$, $R^{9'}$ und die Reste, die nicht für $CH_2$ oder eine Bindung stehen, unabhängig voneinander für Methylreste oder H, bevorzugt für H stehen. Am meisten bevorzugt ist die Ausführungsform a) mit der Einschränkung, dass $R^9$, $R^{9'}$ und die und die Reste, die nicht für $CH_2$ oder eine Bindung stehen, unabhängig voneinander für Methylreste oder H, bevorzugt für H stehen.

Beispiele für besonders bevorzugte Verbindungen der Formel IV sind 3R,3aR,6R,6aR-Hexahydrofuro[3,2-*b*]furan-3,6-diamin, 3R,3aR,6S,6aR-Hexahydrofuro[3,2-*b*]furan-3,6-diamin, 3S,3aR,6S,6aR-Hexahydrofuro[3,2-*b*]furan-3,6-diamin, 3R,3aR,6R,6aR-6-(aminomethyl)-hexa-hydrofuro[3,2-*b* furan-3-yl]-methanamin, 3R,3aR,6S,6aR-6-(aminomethyl)-hexahydrofuro[3,2-*b* furan-3-yl]-methanamin, 3S,3aR,6S,6aR-6-(aminomethyl)-hexahydrofuro[3,2-*b* furan-3-yl]-methanamin und deren Gemische.

Biobasierte Diamine:

Diamine der Formel III, wobei $Y^1$ für Y'b, und

2 der Reste $R^1$ bis $R^4$ für $CH_2$,
und die beiden verbleibenden dieser Reste, sowie $R^{1'}$ bis $R^{4'}$ unabhängig voneinander für organische Reste mit bis zu 18 Kohlenstoffatomen gemäß obiger Beschreibung oder für H, bevorzugt für Alkylreste mit 1 bis 8 Kohlenstoffatomen oder H,
oder $R^3$ und $R^4$ für eine Bindung,
und $R^1$, $R^{1'}$, $R^2$, $R^{2'}$, $R^{3'}$ und $R^{4'}$unabhängig voneinander für organische Reste mit bis zu 18 Kohlenstoffatomen gemäß obiger Beschreibung oder für H, bevorzugt für Alkylreste mit 1 bis 8 Kohlenstoffatomen oder H,

und
Diamine der Formel IV, wobei $Y^2$ und $Y^3$ für $Y^2$a und $Y^3$a,

$R^{10}$ für $CH_2$ oder eine Bindung und
$R^3$ für $CH_2$ oder eine Bindung ,
und $R^1$, $R^2$, $R^{1'}$, $R^{2'}$, $R^{3'}$ und $R^{10'}$ unabhängig voneinander für Methyl-, Ethyl-, Propyl-, iso-Propyl-, Butyl-, iso-Butyl-Reste oder H, bevorzugt für H, und $R^9$, $R^{9'}$ unabhängig voneinander für $CH_3$ oder H, bevorzugt für H,

und im Besonderen Oxacyclopentan-2,5-di-methylenamin (Bis(aminomethyl)tetrahydrofuran / Formel III) und die

oben genannten Hexahydrofuro[3,2-*b*]furan-3,6-diamin- bzw. (Aminomethyl)-hexahydrofuro[3,2-*b* furan-3-yl]-methanamin-Stereoisomere (Formel IV) lassen sich beispeilsweise aus Furfural oder substituierten Furfuralen, wie z.B. 5-Hydroxymethylfurfural, bzw. aus Isosorbid, Isomannid oder Isoidid als Ausgangsstoffen herstellen. Diese Ausgangsstoffe können biobasiert oder nicht-biobasiert erhalten werden.

Der Begriff "Ausgangsstoff" bedeutet, dass der Stoff bei der Herstellung der Diamine chemisch umgewandelt, also verbraucht wird.

Der Begriff "biobasiert" wird im Rahmen der vorliegenden Erfindung dahingehend definiert, dass eine Verbindung, ein Material, oder ähnliches, beispielsweise ein Ausgangsstoff oder ein Polyamin der Formel III oder IV, aus erneuerbaren Quellen, wie Pflanzen, Mikroben, Algen oder Tieren erhalten wurde oder daraus präpariert wurde.

Demgegenüber stehen Verbindungen und Materialien, die aus nicht-erneuerbaren Quellen erhalten oder präpariert wurden. Nicht-erneuerbare Quellen umfassen z.B. fossile Rohstoffe, die in geologischer Vorzeit aus toten Lebewesen entstanden sind. Dies sind insbesondere Erdöl, Braunkohle, Steinkohle, Torf und Erdgas. Verbindungen und Materialien, die aus nicht-erneuerbaren Quellen erhalten oder präpariert wurden, werden im Rahmen der vorliegenden Erfindung als nicht-biobasiert oder "künstlich hergestellt" definiert.

Biobasierte Ausgangsstoffe können unmittelbar aus den oben genannten erneuerbaren Quellen gewonnen werden oder durch Folgereaktionen aus Verbindungen oder Materialien, die aus diesen Quellen erhalten wurden, hergestellt werden.

Werden zur Herstellung der erfindungsgemäßen Polyasparaginsäureester-Zusammensetzungen A1 biobasierte Diamine eingesetzt, so bedeutet dies, dass diese Diamine vollständig oder teilweise biobasiert sind. Dies hängt davon ab, in welchem Umfang bei der Herstellung biobasierte Ausgangsstoffe eingesetzt werden. Bei teilweise biobasierten Diaminen wird zumindest ein biobasierter Ausgangsstoff eingesetzt.

Das oben angesprochene Furfural oder dessen substituierte Derivate, wie z.B. 5-Hydroxymethylfurfural, können biobasiert aus Pentosen und Hexosen erhalten werden, welche wiederum aus der Fraktionierung von Cellulose, Stärke oder anderen Polysacchariden erhältlich sind. Die beschriebene Synthese stellt lediglich ein Beispiel für eine mögliche Synthese dar. Selbstverständlich ist auch jede andere Art der biobasierten Herstellung von Furfural bzw. dessen Derivaten möglich.

Die Herstellung von 2,5-Bis(aminomethyl)tetrahydrofuran kann z.B. durch chemische Umwandlung von biobasiertem 5-Hydroxymethylfurfural über 2,5-Furandicarbonsäure, 2,5-Bis(hydroxymethyl)furan und 2,5-Dihydroxymethyltetrahydrofuran, oder durch chemische Umwandlung von biobasiertem Furfural über Furfurylalkohol, Tetrahydrofurfurylalkohol, 2-Hydroxymethyl-vinyl-furan, 2,5-Bis(hydroxymethyl)furan und 2,5-Dihydroxymethyltetrahydrofuran erfolgen. Auch diese Synthesen sind nur beispielhaft genannt und sind nicht abschließend zu verstehen.

Isosorbid, Isomannid oder Isoidid können biobasiert ebenfalls beispielsweise aus Pentosen und Hexosen erhalten werden. Aus diesen Zuckern wird Sorbit bzw. Mannit hergestellt, die dann wiederum zu Isosorbid bzw. Isomannid dehydratisiert werden. Auch kann Isosorbid zu Isomannid isomerisiert werden. Isoidid wird aus der Isomerisierung von Isosorbid oder Isomannid erhalten. Auch die hier beschriebenen Synthesen sind lediglich Beispiele. Selbstverständlich ist auch jede andere Art der biobasierten Herstellung von Isosorbid, Isomannid oder Isoidid möglich.

Auch sei an dieser Stelle klargestellt, dass die oben genannten Diamine auch nicht-biobasiert erhalten werden können und als solche auch zur Herstellung der erfindungsgemäßen Polyasparaginsäureester-Zusammensetzungen A1 eingesetzt werden können.

In einer Ausführungsform der Erfindung entsprechen Polyasparaginsäureester-haltige Zusammensetzungen A1 solchen, bei denen X erhalten werden kann durch Entfernung der primären Aminogruppen aus cyclischen Ethern der oben beschriebenen Art, die auf Ausgangstoffen basieren, die biobasiert erhalten wurden.

In einer bevorzugten Variante dieser Ausführungsform entsprechen Polyasparaginsäureester-haltige Zusammensetzungen A1 solchen, bei denen X erhalten werden kann durch Entfernung der primären Aminogruppen aus cyclischen Ethern, die

Diaminen der Formel III, wobei $Y^1$ für Y'b, und

2 der Reste $R^1$ bis $R^4$ für $CH_2$,
und die beiden verbleibenden dieser Reste, sowie $R^{1'}$ bis $R^{4'}$ unabhängig voneinander für organische Reste mit bis zu 18 Kohlenstoffatomen gemäß obiger Beschreibung oder für H, bevorzugt für Alkylreste mit 1 bis 8 Kohlenstoffatomen oder H,
oder $R^3$ und $R^4$ für eine Bindung,
und $R^1$, $R^{1'}$, $R^2$, $R^{2'}$, $R^{3'}$ und $R^{4'}$unabhängig voneinander für organische Reste mit bis zu 18 Kohlenstoffatomen gemäß obiger Beschreibung oder für H, bevorzugt für
Alkylreste mit 1 bis 8 Kohlenstoffatomen oder H,

oder

Diaminen der Formel IV, wobei $Y^2$ und $Y^3$ für $Y^2a$ und $Y^3a$,

$R^{10}$ für $CH_2$ oder eine Bindung und
$R^3$ für $CH_2$ oder eine Bindung ,
und $R^1$, $R^2$, $R^{1'}$, $R^{2'}$, $R^{3'}$ und $R^{10'}$ unabhängig voneinander für Methyl-, Ethyl-, Propyl-, iso-Propyl-, Butyl-, iso-Butyl-Reste oder H, bevorzugt für H, und $R^9$, $R^{9'}$ unabhängig voneinander für $CH_3$ oder H, bevorzugt für H,

entsprechen, wobei diese Diamine auf Ausgangstoffen basieren, die biobasiert erhalten wurden.

In einer besonders bevorzugten Variante dieser Ausführungsform entsprechen Polyasparaginsäureester-haltige Zusammensetzungen A1 solchen, bei denen X erhalten werden kann durch Entfernung der primären Aminogruppen aus 3R,3aR,6R,6aR-Hexahydrofuro[3,2-*b*]furan-3,6-diamin, 3R,3aR,6S,6aR-Hexahydrofuro[3,2-*b*]furan-3,6-diamin, 3S,3aR,6S,6aR-Hexahydrofuro[3,2-*b*]furan-3,6-diamin, 3R,3aR,6R,6aR-6-(aminomethyl)-hexa-hydrofuro[3,2-*b* furan-3-yl]-methanamin, 3R,3aR,6S,6aR-6-(aminomethyl)-hexahydrofuro[3,2-*b* furan-3-yl]-methanamin, 3S,3aR,6S,6aR-6-(aminomethyl)-hexahydrofuro[3,2-*b* furan-3-yl]-methanamin oder Oxacyclopentan-2,5-di-methylenamin, wobei diese Diamine auf Ausgangstoffen basieren, die biobasiert erhalten wurden.

Sofern die Zusammensetzung A1 einen oder mehrere Polyasparaginsäureester der allgemeinen Formel (II) enthält, so ist dieser/ sind diese mit einem Anteil von > 0%, bevorzugt mindestens 0,1% (≥ 0,1%), besonders bevorzugt mindestens 1% (≥ 1%), ganz besonders bevorzugt mindestens 4% (≥ 4%), und bevorzugt von maximal 20% (≤ 20%), besonders bevorzugt maximal 15% (≤ 15%) der GC Oberfläche (gemessen als Flächen-% im Gas Chromatogram) enthalten, wobei die Summe der GC Oberflächen der Verbindungen der beiden allgemeinen Formeln (I) und (II) 100% beträgt. Die angegebenen Ober- und Untergrenzen sind beliebig kombinierbar. Es gelten alle möglichen Kombinationen als offenbart.

Bevorzugt enthalten oder bestehen die Zusammensetzungen A1 einen/aus einem oder mehrere/aus mehreren Polyasparaginsäureester/n der allgemeinen Formel (I) und gegebenenfalls Formel(II), die eine Platin-Cobalt-Farbzahl ≤ 100, besonders bevorzugt ≤ 50 aufweisen. Die Messung der Platin-Cobalt-Farbzahl erfolgt gemäß DIN EN ISO 6271:2016-05.

Gegenstand der vorliegenden Erfindung ist auch ein Verfahren zur Herstellung von Zusammensetzungen A1, enthaltend oder bestehend aus einen/einem oder mehrere/n Polyasparaginsäureester/n der allgemeinen Formel (I) und ggf. Formel (II).

Zusammensetzungen A1, enthaltend oder bestehend aus einen/einem oder mehrere/n Polyasparaginsäureester/n der allgemeinen Formel (I) und Formel (II), lassen sich durch das folgende Verfahren herstellen:

Umsetzung von Polyaminen der allgemeinen Formel (V)

$$X\left[NH_2\right]_m \qquad (V),$$

wobei X

für einen m-wertigen organischen Rest steht, wie er durch Entfernung der primären Aminogruppen aus einem entsprechenden cyclischen Ether, bei dem es sich um einen Monocyclus oder Polycyclus handelt, der im Ring bzw. den Ringen gesättigte und/oder ungesättigte Kohlenstoff-Kohlenstoff-Bindungen aufweist und der an wenigstens 2 der Ringkohlenstoffatome eine primäre Aminogruppe und/oder eine aliphatisch gebundene primäre Aminogruppe aufweist, wobei

m für eine ganze Zahl >1, bevorzugt für 3, besonders bevorzugt für 2 steht, mit Verbindungen der allgemeinen Formel (VI)

R1OOC-CH=CH-COOR2 (VI),

wobei R1 und R2

für gleiche oder verschiedene organische Reste, vorzugsweise für gleiche oder verschiedene Alkylreste mit je 1 bis 18 Kohlenstoffatomen, besonders bevorzugt für gleiche oder verschiedene Alkylreste mit je 1 bis 8 Kohlenstoffatomen, ganz besonders bevorzugt für jeweils Alkylreste wie Methyl-, Ethyl-, Propyl-, iso-Propyl-, Butyl- oder iso-Butyl-Reste und am meisten bevorzugt für Ethyl stehen.

Zur Herstellung der Zusammensetzung A1, enthaltend oder bestehend aus einen/einem oder mehrere/n Polyasparaginsäureester/n der allgemeinen Formel (I) und Formel (II) werden die Verbindungen der allgemeinen Formel (V) und (VI) bei Temperaturen zwischen 0°C und 100°C, vorzugsweise 20° bis 80°C und besonders bevorzugt 20° bis 60°C, in einem Verhältnis der Äquivalente der primären Aminogruppen der Verbindungen der allgemeinen Formel (V) zu den C=C-Doppelbindungsäquivalenten der Verbindungen der allgemeinen Formel (VI) von 1:1.2 bis 1.2:1, vorzugsweise jedoch 1:1.05 bis 1.05:1 bis zu einem Restgehalt an Verbindungen der allgemeinen Formel (VI) von 2 bis 15 Gew.-Prozent, bevorzugt von 3 bis 10 Gew.-Prozent umgesetzt.

Zusammensetzungen A1, die nur Polyasparaginsäureester der allgemeinen Formel (I) enthalten, nicht jedoch der Formel (II), lassen sich durch analog herstellen, wobei jedoch mit einem Überschuss an Verbindungen der allgemeinen Formel (VI) gearbeitet wird, d.h. in einem Verhältnis der Äquivalente der primären Aminogruppen der Verbindungen der allgemeinen Formel (V) zu den C=C-Doppelbindungsäquivalenten der Verbindungen der allgemeinen Formel (VI) von 1:10, bevorzugt 1:5, besonders bevorzugt 1:2.

Dem oben beschriebenen Verfahren kann sich ein Destillationsschritt zur Entfernung des nicht umgesetzten Anteils an der Verbindung der allgemeinen Formel (VI) anschließen. Eine solche Vorgehensweise ist bevorzugt.

Bevorzugt sind daher Zusammensetzungen A1 enthaltend oder bestehend aus einen/einem oder mehrere/n Polyasparaginsäureester/n der allgemeinen Formeln (I) und gegebenenfalls Formel (II), mit einem Anteil von 0,01 bis 1,2 Gew.-% (≥ 0,01 bis ≤ 1,2 Gew.-%), bevorzugt 0,01 bis 1 Gew.- % (≥ 0,01 bis ≤ 1 Gew.-%), besonders bevorzugt 0,01 bis 0,1 Gew.-% (≥ 0,01 bis ≤ 0,1 Gew.-%), bezogen auf das Gesamtgewicht der Komponente A, an Verbindungen der Formel (VI), wobei es sich bevorzugt um Fumarsäuredialkylester handelt.

Geeignete Bedingungen während der Destillation sind ein Druckbereich zwischen 0.01 und 2 mbar und eine Temperatur des Sumpfablaufs beim Austritt aus der Destillationsapparatur ≤ 170°C und ≥ der Temperatur liegt, die sich aus der folgenden Formel (VII) ergibt:

$$T(\text{Sumpfablauf}) = 27 \times \ln(p) + 150 \qquad \text{(VII)}$$

wobei

| | |
|---|---|
| T(Sumpfablauf) | für die Temperatur des Sumpfablaufs in °C und |
| p | für den Druck in der Destillationsapparatur in mbar stehen. |

Durch Einhalten dieses Druckbereichs wird gewährleistet, dass einerseits moderate Temperaturen im Sumpfablauf ausreichen, um die Fumarsäuredialkylester im gewünschten Umfang abzureichern, andererseits das Verfahren im technischen Maßstab anwendbar bleibt. Bei geringerem Druck wird die Gasdichte zu gering und die erforderlichen Apparate deshalb so groß, dass das Verfahren aus wirtschaftlicher Sicht nachteilig wird.

Bevorzugt ist die Temperatur des Sumpfablaufs ≤ 170°C, dabei jedoch mindestens 20 K oberhalb der Temperatur, die sich aus der Formel (VII) ergibt, besonders bevorzugt liegt sie zwischen 20 K und 40 K oberhalb der Temperatur, die sich aus der Formel (VII) ergibt jedoch nicht oberhalb von 170°C.

Primäre Polyamine der allgemeinen Formel (V), die in dem oben beschriebenen Verfahren Anwendung finden, entsprechenden den monocyclischen und polycyclischen Ethern, die im Rahmen der Diskussion der Formeln I und II beschrieben wurden, einschließlich der dort beschriebenen Vorzugsbereiche.

Bevorzugte Verbindungen der allgemeinen Formel (VI), die in dem oben beschriebenen Verfahren Anwendung finden sind Malein- oder Fumarsäureester der allgemeinen Formel (VI), worin R1 und R2 für gleiche oder verschiedene organische Reste mit je 1 bis 18 Kohlenstoffatomen stehen. Bevorzugt stehen R1 und R2 unabhängig voneinander für lineare oder verzweigte Alkylreste mit 1 bis 8 Kohlenstoffatomen, besonders bevorzugt für jeweils Alkylreste wie Methyl-, Ethyl-, Propyl-, iso-Propyl-, Butyl- oder iso-Butyl-Reste und ganz besonders bevorzugt für Ethyl.

Beispielhaft für Verbindungen der allgemeinen Formel (VI) seien die folgenden Verbindungen genannt: Maleinsäuredimethylester, -diethylester, -di-n oder -iso-propylester, -di-n-butylester, -di-2-ethyl-hexylester oder die entsprechenden Fumarsäureester. Ganz besonders bevorzugt ist Maleinsäurediethylester.

Ein Gegenstand der vorliegenden Erfindung sind auch die Polyasparaginsäureester-enthaltenden oder daraus bestehenden Zusammensetzungen A1 in Mischung mit weiteren, von A1 verschiedenen Polyasparaginsäureestern oder Polyasparaginsäureester-enthaltenden bzw. daraus bestehenden Zusammensetzungen (Komponente A2).

Zu diesen Zusammensetzungen A2 gehören z.B. die im Folgenden beschriebenen Zusammensetzungen A2.1, mit denen die Zusammensetzungen A1 in einem gewissen Rahmen abgemischt werden können:
Zusammensetzungen A2.1 enthaltend oder bestehend aus einen/einem oder mehrere/n Polyasparaginsäureester/n der allgemeinen Formel (VIII)

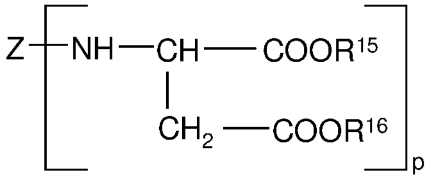

(VIII),

in welcher

| | |
|---|---|
| Z | für einen p-wertigen, gegebenenfalls ein oder mehrere Heteroatome enthaltenden organischen Rest steht, wie er durch Entfernung der primären Aminogruppen aus einem entsprechenden, (cyclo)aliphatisch bzw. araliphatisch gebundene primäre Aminogruppen aufweisenden Polyamin des Molekulargewichtsbereichs 60 bis 6000 g/mol erhalten werden kann, und der weitere, gegenüber Isocyanatgruppen reaktive und/oder bei Temperaturen bis 100°C inerte funktionelle Gruppen enthalten kann, |
| $R^{15}$ und $R^{16}$ | für gleiche oder verschiedene organische Reste, mit je 1 bis 18 Kohlenstoffatomen stehen, |
| p | für eine ganze Zahl > 1 steht, bevorzugt für 2, |

und
gegebenenfalls einen/einem oder mehrere/n Polyasparaginsäureester/n mit primärer Aminogruppe der allgemeinen Formel (IX)

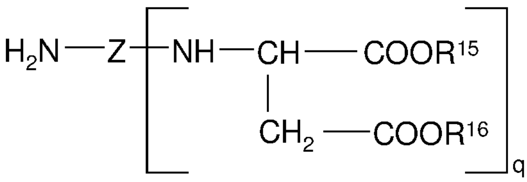

(IX)

in welcher

| | |
|---|---|
| q | für p-1 steht, |
| Z, Reste $R^{15}$ und $R^{16}$ | die oben genannten Bedeutungen haben. |

Bevorzugt sind hierbei Polyasparaginsaureester-haltige Zusammensetzungen A2.1 enthaltend oder bestehend aus einen/einem oder mehrere/n Polyasparaginsäureester/n der allgemeinen Formeln (VIII) und gegebenenfalls (IX) bei denen $R^{15}$ und $R^{16}$ für gleiche oder verschiedene Alkylreste mit je 1 bis 18 Kohlenstoffatomen, bevorzugt gleiche oder verschiedene Alkylreste mit je 1 bis 8 Kohlenstoffatomen und ganz besonders bevorzugt für jeweils Alkylreste wie Methyl-, Ethyl-, Propyl-, iso-Propyl-, Butyl- oder iso-Butyl-Reste stehen. Am meisten bevorzugt ist Ethyl.

Polyasparaginsaureester-haltige Zusammensetzungen A2.1 sind Zusammensetzungen enthaltend oder bestehend aus einen/einem oder mehrere/n Polyasparaginsäureester/n der allgemeinen Formeln (VIII) und gegebenenfalls (IX), bei denen Z für organische Reste steht, die durch Entfernung der primären Aminogruppen aus einem entsprechenden, (cyclo)aliphatisch bzw. araliphatisch gebundene primäre Aminogruppen aufweisenden Polyamin erhalten werden. Beispielhaft seien die folgenden Polyamine genannt: Ethylendiamin, 1,2-Diaminopropan, 1,4-Diaminobutan, 1,5-Diaminopentan, 2,5-Diamino-2,5-dimethylhexan, 1,5-Diamino-2-methylpentan (DytekOA, Fa DuPont), 1,6-Diaminohexan, 2,2,4- und/oder 2,4,4-Trimethyl-1,6-diaminohexan, 1,11-Diaminoundecan, 1,12-Diaminododecan oder Triaminononan, Etheramine, wie z.B. 4,9-dioxadodecane-1,12-diamine, 4,7,10-trioxatridecane-1,13-diamine, oder höhermolekulare Polyetherpolyamine mit aliphatisch gebundenen, primären Aminogruppen, wie sie beispielsweise unter der Bezeichnung Jeffamin® von der Firma Huntsman vertrieben werden. Ebenfalls einsetzbar sind aliphatische polyzyklische Polyamine, wie Tricyclodecanbismethylamin (TCD-Diamin) oder Bis(aminomethyl)norbornane, amino-funktionelle Siloxane, z.B. Diaminopropylsiloxan G10 DAS (Fa. Momentive), Fettalkyl-basierte Amine, wie z.B. Fentamine der Fa. Solvay, Dimerfettsäurediamine wie z.B Priamine der Fa. Croda.

Weitere Beispiele für einsetzbare Diamine sind 1-Amino-3,3,5-trimethyl-5-aminomethylcyclohexan (IPDA), 2,4- und/oder 2,6-Hexahydrotoluylendiamin (H6-TDA), Isopropyl-2,4-diaminocyclohexan, und/oder Isopropyl-2,6-diaminocyclohexan, 1,3-Bis-(aminomethyl)-cyclohexan, 2,4'-, und/oder 4,4'-Diamino-dicyclohexylmethan, 3,3'-Dimethyl-4,4'-diamino-dicyclohexylmethan (Laromin® C 260, Fa. BASF AG), die isomere, eine Methylgruppe als Kernsubstituenten aufweisende Diaminodicyclohexylmethane (=C-Monomethyldiaminodicyclohexylmethane), 3(4)-Aminomethyl-1-methylcyclohexylamin (AMCA) sowie araliphatische Diamine, wie z.B. 1,3-Bis-(aminomethyl)-benzol oder m-Xylylendiamin.

Bevorzugte Amine sind: Polyetherpolyamine mit aliphatisch gebundenen primären Aminogruppen, 1,2 Diaminopropan, 1,4-Diaminobutan, 1,6-Diaminohexan, 1,5-Diamino-2-methylpentan, 2,5 Diamino-2,5-dimethylhexan, 2,2,4- und/oder 2,4,4-Trimethyl-1,6-diaminohexan, 1,11-Diaminounodecan, 1,12-Diaminododecan, 1-Amino-3,3,5-trimehtyl-5-aminomethylcyclohexan, 2,4- und/oder 2,6-Hexahydrotoluylendiamin, 1,5-Diaminopentan, 2,4'- und/oder 4,4'-Diamino-dicyclohexylmethan oder 3,3'-Dimethyl-4,4'-diamino-dicyclohexylmethan. Besonders bevorzugt sind 1,2 Diaminopropan, 1,4-Diaminobutan, 1,5-Diaminopentan, 1,6-Diaminohexan, 1,5-Diamino-2-methylpentan, 2,5 Diamino-2,5-dimethylhexan, 2,2,4- und/oder 2,4,4-Trimethyl-1,6-diaminohexan, 1,11-Diaminounodecan, 1,12-Diaminododecan, 1-Amino-3,3,5-trimehtyl-5-aminomethylcyclohexan, 2,4- und/oder 2,6-Hexahydrotoluylendiamin, 2,4'- und/oder 4,4'-Diamino-dicyclohexylmethan oder 3,3'-Dimethyl-4,4'diamino-dicyclohexylmethan. Ganz besonders bevorzugt sind 3,3'-Dimethyl-4,4'diaminodicyclohexylmethan, 2,4'- und/oder 4,4 '-Diaminodicyclohexylmethan, 1,5-Diamino-2-methylpentan.

Die Herstellung der Zusammensetzung A2.1 kann unter Verwendung der oben angegebenen Amine analog dem zuvor für Zusammensetzung A1 beschriebenen Herstellverfahren erfolgen und ist darüber hinaus auch beispielsweise in der EP19158880.5 oder EP19158883.9 offenbart.

Für eine Mischung aus Zusammensetzung A1 und A2.1 gilt:
Der Anteil der Verbindungen der Formel VIII bzw. der Formel VIII und IX (Gesamtmenge, falls Verbindungen der Formel IX vorhanden) an dem Gesamtgewicht der Verbindungen der Formeln I, II (falls vorhanden), VIII und IX (fallsvorhanden) beträgt ≤ 20 Gew.-%, bevorzugt ≤ 15 Gew.-%, besonders bevorzugt ≤ 10 Gew.-%.

Gegenstände der vorliegenden Erfindung sind auch die Verwendung von Zusammensetzungen A1, enthaltend einen oder mehrere Polyasparaginsäureester der allgemeinen Formel (I) und gegebenenfalls Formel (II), gegebenenfalls in Mischung mit weiteren Polyasparaginsäureestern oder Polyasparaginsäureester-enthaltenden bzw. daraus bestehenden Zusammensetzungen A2, zur Herstellung von Beschichtungszuammensetzungen, bevorzugt zweikomponentigen Beschichtungszusammensetzungen (2K-Beschichtungsmittel), die so erhältlichen Beschichtungsmittel, deren Verwendung zur Beschichtung von Substraten, das Verfahren zum Beschichten eines Substrats und die so erhältlichen Substrate selbst.

Gegenstand der vorliegenden Erfindung sind daher Beschichtungszusammensetzungen, bevorzugt zweikomponentige Beschichtungszusammensetzungen (2K-Beschichtungsmittel), enthaltend

a1) wenigstens eine Polyasparaginsäureester-haltige Zusammensetzung A1,
a2) gegebenenfalls weitere, von A1 verschiedene Polyasparaginsäureester oder Polyasparaginsäureester-enthaltende bzw. daraus bestehende Zusammensetzungen A2,
b) mindestens eine Polyisocyanatkomponente B
c) gegebenenfalls eine oder mehrere, von A1 und A2 verschiedene, gegenüber Isocyanatgruppen reaktionsfähige Komponenten C,
d) gegebenenfalls Hilfs- und Zusatzstoffe (Komponente D).

Die erfindungsgemäßen, bevorzugt zweikomponentigen Beschichtungszusammensetzungen enthalten mindestens eine Polyisocyanatkomponente B.

Geeignete Polyisocyanatkomponenten B sind organische Polyisocyanate mit einer mittleren NCO-Funktionalität von mindestens 2 und einem Molekulargewicht von mindestens 140 g/mol. Gut geeignet sind vor allem unmodifizierte organische Polyisocyanate des Molekulargewichtsbereichs 140 bis 300 g/mol, Lackpolyisocyanate eines Molekulargewichts im Bereich von 300 bis 1000 g/mol, sowie Urethan-, Harnstoff- und / oder Allophanatgruppen- aufweisende NCO-Prepolymere eines über 400 g/mol liegenden Molekulargewichtes oder deren Gemische.

Unter dem Begriff "Lackpolyisocyanate" sind im Sinne der Erfindung Verbindungen oder Gemische von Verbindungen zu verstehen, die durch an sich bekannte Oligomerisierungsreaktion von einfachen Polyisocyanaten erhalten werden können. Geeignete Oligomerisierungsreaktionen sind z.B. die Carbodiimidisierung, Dimerisierung, Trimerisisierung, Biuretisierung, Harnstoffbildung, Urethanisierung, Allophanatisierung und/oder Cyclisierung unter Ausbildung von Oxadiazinstrukturen. Bei Oligomerisierung können mehrere der genannten Reaktionen gleichzeitig oder nacheinander ablaufen.

Bevorzugt handelt es sich bei den "Lackpolyisocyanaten" um Biuretpolyisocyanate, Isocyanuratgrupen-aufweisende Polyisocyanate, Isocyanurat- und Uretdiongruppen-aufweisende Polyisocyanatgemische, Urethan- und/oder Allophanatgruppen-aufweisende Polyisocyanate oder um Isocyanurat- und Allophanatgruppen-aufweisende Polyisocyanatgemische auf Basis einfacher organischer Polyisocyanate.

Ebenfalls geeignet als Polyisocyanatkomponente B sind die an sich bekannten Isocyanatgruppen aufweisenden Prepolymere auf Basis einfacher organischer Polyisocyanate und/oder auf Basis von Lackpolyisocyanaten einerseits und organischen Polyhydroxyverbindungen eines über 300 g/mol liegenden Molekulargewichts andererseits. Während es sich bei den Urethangruppen-aufweisenden Lackpolyisocyanaten um Derivate von niedermolekularen Polyolen des Molekulargewichtsbereichs von 62 bis 300 g/mol handelt, geeignete Polyole sind beispielsweise Ethylenglykol, Propylenglykol, Trimethylolpropan, Glycerin oder Gemische dieser Alkohole, werden zur Herstellung der Isocyanatgruppen aufweisenden Prepolymeren Polyhydroxyverbindungen eines über 300 g/mol, bevorzugt über 400 g/mol, besonders bevorzugt eines zwischen 400 und 8000 g/mol liegenden Molekulargewichts eingesetzt. Derartige Polyhydroxylverbindungen sind insbesondere solche, die pro Molekül 2 bis 6, bevorzugt 2 bis 3 Hydroxylgruppen aufweisen und aus der Gruppe, bestehend aus Ether-, Ester-, Thioether-, Carbonat- und Polyacrylatpoloyolen und Gemischen aus derartigen Polyolen ausgewählt sind.

Bei der Herstellung der Isocyanatgruppen aufweisenden Prepolymeren können die genannten höhermolekularen Polyole auch in Abmischungen mit den genannten niedermolekularen Polyolen zur Anwendung gelangen, so dass unmittelbar Gemische aus niedermolekularen, Urethangruppen aufweisenden Lackpolyisocyanaten und höhermolekularen NCO-Prepolymeren resultieren, die ebenfalls als erfindungsgemäße Polyisocyanatkomponente b) geeignet sind.

Zur Herstellung der Isocyanatgruppen aufweisenden Prepolymeren oder deren Gemischen mit den Lackpolyisocyanaten werden einfache organische Polyisocyanate der nachstehend beispielhaft genannten Art oder Lackpolyisocynate mit den höhermolekularen Hydroxylverbindungen oder deren Gemischen mit niedermolekularen Polyhydroxylverbindungen der beispielhaft genannten Art unter Einhaltung eines NCO/OH Aquivalentverhaltnisses von 1.1:1 bis 40:1, bevorzugt 2:1 bis 25:1 unter Urethan und / oder Allophanatbildung umgesetzt. Gegebenenfalls kann bei Verwendung eines Überschusses an destillierbarem einfachem organischem Polyisocyanat dieser im Anschluss an die Umsetzung destillativ entfernt werden, so dass monomerenfreie Isocyanatgruppen aufweisende NCO-Prepolymere vorliegen, die ebenfalls als Polyisocyanatkomponente b) eingesetzt werden können.

Beispiele für geeignete einfache organische Polyisocyanate sind 1,4-Diisocyanatobutan, 1,5-Diisocyanatopentan, 1,6-Diisocyanatohexan (HDI), 1,5-Diisocyanato-2,2-dimethylpentan, 2,2,4-bzw. 2,4,4-Trimethyl-1,6-diisocyanatohexan, Tetramethylxylylendiisocyanat (TMXDI) 1-Isocyanato-3,3,5-trimethyl-5-isocyanatomethylcyclohexan (Isophorondiisocyanat, IPDI), 1-Isocyanato-1-methyl-4-(3)-isocyanatomethylcyclohexan, Dicyclohexylmethan-2,4'-und/oder 4,4'-diisocyanat, 1,10-Diisocyanatodecan, 1,12-Diisocyanatododecan, Cyclohexan-1,3- und -1,4-diisocyanat, Xylylendiisocyanat-Isomere, Triisocyanatononan (TIN), Naphthylen-1,5-diisocyanat, 2,4-Diisocyanatoluol oder dessen Gemische mit 2,6-Diisocyanatotoluol mit bevorzugt, bezogen auf Gemische, bis zu 35 Gew.-% 2,6-Diisocyanatotoluol, 2,2'-, 2,4'-, 4,4'-, Diisocyanatodiphenylmethan oder technische Polyisocyanatgemische der Diphenylmethanreihe, oder beliebige Gemische der genannten Polyisocyanate.

Bevorzugt kommen dabei aliphatische, cycloaliphatische oder araliphatische Polyisocyanate, ausgewählt aus der Gruppe 1,4-Diisocyanatobutan, 1,5-Diisocyanatopentan, 1,6-Diisocyanatohexan (HDI), 1,5-Diisocyanato-2,2-dimethylpentan, 2,2,4- bzw. 2,4,4-Trimethyl-1,6-diisocyanatohexan, Tetramethylxylylendiisocyanat (TMXDI) 1-Isocyanato-3,3,5-trimethyl-5-isocyanatomethylcyclohexan (Isophorondiisocyanat, IPDI), 1-Isocyanato-1-methyl-4-(3)-isocyanatomethylcyclohexan, Dicyclohexylmethan-2,4'-und/oder 4,4'-diisocyanat, 1,10-Diisocyanatodecan, 1,12-Diisocyanatododecan, Cyclohexan-1,3- und -1,4-diisocyanat, Xylylendiisocyanat-Isomere, Triisocyanatononan (TIN), oder beliebige Gemische derartiger Polyisocyanate, zum Einsatz.

Prinzipiell ist natürlich auch der Einsatz von Mischungen verschiedener Polyisocyanatkomponenten der vorstehend genannten Art möglich.

Neben den Polyasparaginsäureester-haltigen Zusammensetzungen A1 und ggf. A2 kann die erfindungsgemäße, bevorzugt zweikomponentige Beschichtungszusammensetzung weitere gegenüber Isocyanatgruppen reaktionsfähige Komponenten (Komponenten C) enthalten.

Dies können beispielsweise niedermolekulare Polyole des Molekulargewichtsbereichs von 62 bis 300 g/mol, beispielsweise Ethylenglykol, Propylenglykol, Trimethylolpropan, Glycerin oder Gemische dieser Alkohole, oder Polyhydroxyverbindungen eines über 300 g/mol, bevorzugt über 400 g/mol, besonders bevorzugt eines zwischen 400 und 8000 g/mol liegenden Molekulargewichts sein. Derartige Polyhydroxylverbindungen sind insbesondere solche, die pro Molekül 2 bis 6, bevorzugt 2 bis 3 Hydroxylgruppen aufweisen und aus der Gruppe, bestehend aus Ether-, Ester-, Thioether-, Carbonat- und Polyacrylatpoloyolen und Gemischen aus derartigen Polyolen ausgewählt sind.

Weiterhin können die erfindungsgemäßen, bevorzugt zweikomponentigen Beschichtungszusammensetzung Hilfs- und Zusatzstoffe (Komponente D) enthalten. Dabei handelt es sich um die in der Beschichtungstechnologie üblichen Hilfs- und Zusatzstoffe, wie anorganische oder organische Pigmente, weitere organische Lichtschutzmittel, Radikalfänger, Lackadditive, wie Dispergier-, Verlauf-, Verdickungs-, Entschäumungs- und andere Hilfsmittel, Haftmittel, Fungizide, Bakterizide, Stabilisatoren oder Inhibitoren, Katalysatoren und Lösemittel.

Vorzugsweise beträgt das Verhältnis der Isocyanatgruppen der Polyisocyanatkomponente B zu den isocyanatreaktiven Gruppen aus den Komponenten A1, A2 und C 0,5 zu 1,0 bis 3,0 zu 1,0. Besonders bevorzugt ist ein Verhältnis von 0,9 zu 1,0 bis 1,5 zu 1,0. Ganz besonders bevorzugt ist ein Verhältnis von 1,05 zu 1,0 bis 1,25 zu 1,0.

Die erfindungsgemäße Beschichtungszusammensetzung ist vorzugsweise keine aufschäumbare oder schaumbildende Zusammensetzung. Die Zusammensetzung ist vorzugsweise nicht radikalisch polymerisierbar, insbesondere nicht photo-polymerisierbar, d.h. die Zusammensetzung härtet nicht durch radikalische Prozesse aus, insbesondere nicht durch radikalische Polymerisationsprozesse die durch aktinische Strahlung initiiert werden.

Die Herstellung der erfindungsgemäßen Beschichtungszusammensetzungen erfolgt nach in der Technologie

der Lacke und Beschichtungen an sich bekannten Methoden.

Eine bevorzugte Herstellmethode für eine zweikomponentige Beschichtungszusammensetzung ist im Folgenden beschrieben:

Eine isocyanatreaktive (R) und eine isocyanathaltige Komponente (H) werden zunächst getrennt durch Vermischen der jeweiligen isocyanatreaktiven Komponenten A1, ggf. A2 und ggf. C, bzw. durch Vermischen der jeweiligen Polyisocyanatkomponenten B hergestellt. Die Hilfs- und Zusatzstoffe D1 und D2 werden bevorzugt der isocyanatreaktiven Komponente R beigemischt. Ein Vermischen der so hergestellten Komponenten R und H erfolgt erst unmittelbar vor oder während der Applikation. Erfolgt das Vermischen vor der Applikation ist zu beachten, dass die Reaktion der Bestandteile nach dem Vermischen bereits startet. Je nach Auswahl der Komponenten und der Zusatzstoffe wird die Reaktion unterschiedlich schnell ablaufen. Die Verarbeitungszeit innerhalb derer die Zusammensetzung appliziert werden muss, auch als Topfzeit bezeichnet und definiert als die Zeit von der Vermischung der Komponenten bis zur Verdoppelung der Ausgangsviskosität und/oder der Auslaufzeit (bestimmt nach DIN EN ISO 2431:2012-03, jedoch unter Verwendung eines DIN 4 Auslaufbechers), liegt dann je nach Auswahl der Komponenten im Bereich von 1 Minute bis zu 24 Stunden. Die Bestimmung der Topfzeit erfolgt nach dem Fachmann bekannten Methoden.

Die Erfindung betrifft auch ein Verfahren zum Beschichten eines Substrats, welches mindestens folgende Schritte umfasst:

i) Aufbringen der erfindungsgemäßen, bevorzugt zweikomponentigen Beschichtungszusammensetzung auf mindestens einen Teil eines zu beschichtenden Substrats und
ii) Härten der Beschichtungszusammensetzung aus Schritt i).

Die Substrate können bereits mit einer oder mehreren Lackschichten ganz oder teilweise beschichtet sein. Diese Lackschichten können noch ungehärtet oder feucht, teilgehärtet oder vollständig gehärtet sein, vorzugsweise sind die weiteren Lackschichten auf dem Substrat teilgehärtet oder vollständig gehärtet. Beispiele für Lackschichten sind Grundierungen, Primer, Füller, Spachtel, Basislacke oder bereits vollständig lackierte Substrate, die nach einer eventuellen Vorbehandlung wie Anschleifen oder Plasmaaktivierung erneut überschichtet werden.

Die erfindungsgemäßen Beschichtungsmittel kommen vorzugsweise in den Bereichen Korrosionschutz, Autoerstlackierung, Autoreparaturlackierung, Großfahrzeuglackierung, Kunststofflackierung, allgemeine Industrielackierung, Bodenbeschichtung und/oder Holz-/Möbellackierung zur Anwendung.

## Experimenteller Teil

### Rohstoffe und Substrate:

Desmophen NH 1220: ein aminofunktioneller Reaktionspartner mit einer Aminzahl von 240-248 mg KOH/g, einer Viskosität (25 °C) von ≤ 100mPa·s und eine Farbzahl (Hazen) von ≤ 250, Hersteller Fa. Covestro.

Desmophen NH 1420: ein aminofunktioneller Reaktionspartner mit einer Aminzahl von 199-203 mg KOH/g, einer Viskosität (25 °C) von 900 - 2.000 mPa·s und eine Farbzahl (Hazen) von ≤ 250, Hersteller Fa. Covestro.

Desmodur N 3900: ein niedrigviskoses HDI-Trimerisat mit ca. 23,5% NCO, eine Viskosität (23 °C) von ca. 730 mPa·s und ≤ 0,25% freies HDI, Hersteller Fa. Covestro.

Lösemittel: Solvesso 100, 1-Methoxy-2-propylacetat (MPA), Ethylacetat (EA), Butylacetat (BA), Aceton (Ac) und Xylol (Xy), Azelis, Deutschland.

Tetrahydrofurandimethanamin Merck, Deutschland.

3S,3aR,6S,6aR-Hexahydrofuro[3,2-h]furan-3,6-diamin Merck, Deutschland.

3S,3aR,6S,6aR-6-(aminomethyl)-hexahydrofuro[3,2-*b* furan-3-yl]-methanamin Merck, Deutschland.

Maleinsäurediethylester: Fa. Aldrich, Deutschland.

Das 3R,3aR,6S,6aR-Hexahydrofuro[3,2-b]furan-3,6-diamin wurde hergestellt nach dem in ChemSusChem 2011, 4, 1823 - 1829 beschriebenen Verfahren.

### Methoden:

Fumarsäurediethylester-Gehalte wurden quantitativ mit Hilfe einer GC-Methode mit einem internen Standard bestimmt. Es wurde ein 6890 Gaschromatograph der Fa. Agilent mit einer Standard-GC-Kapillare (100% Polysiloxan-Phase) und einem FID Detektor verwendet. Die Temperatur des Injektors (Splitausgang) war 180°C, als Trägergas wurde Helium verwendet. Die Bestimmungsgrenze diese Methode betrug 300 ppm.

GC-MS Messungen wurden mit einem 6890 Gaschromatograph und Massen Spektrum Detektor 5973 der Fa. Agilent mit Standard-Ionisierung (electron impact) mit 70eV, eine Standard-GC-Kapillare (100% Polysiloxan-Phase) und Split-Injektion bei 250°C Injektortemperatur durchgeführt. Ausgewertet wurden die % Flächen des Gaschromatograms.

Sämtliche Viskositatsmessungen erfolgten mit einem Physica MCR 51 Rheometer der Fa. Anton Paar Germany GmbH (Deutschland) nach DIN EN ISO 3219:1994-10 bei 23°C.

Die Hazen-Farbzahlen wurden an einem LICO 400 Farbmessgerät der Fa. Hach Lange GmbH (Deutschland), gemäß DIN EN ISO 6271:2016-05 ermittelt.

Die Bestimmung der Aminzahlen erfolgte titrimetrisch nach EN ISO 9702:1998 (Perchlorsäre-Methode) mit der Ausnahme, dass die Ergebnisse als Aminzahl angegeben wurden. Die Aminzahl in mg KOH/g wurde nach folgender Gleichung berechnet:

$$Aminzahl = \frac{(a-b)x\,5{,}61}{E}$$

a: der Verbrauch, in Milliliter, an Perchlorsäure, Konzentration c = 0,1 mol/l (im Faktor 5,61 enthalten), im Hauptversuch,;
b: der Verbrauch, in Milliliter, an Perchlorsäure, Konzentration c = 0,1 mol/l (im Faktor 5,61 enthalten), im Blindversuch;
E: die Einwaage, in Gramm.

Die Gelzeit wurde mittels Tecam Gelierzeitmessgerät der Fa. Techne Corp. bestimmt. Diese Methode dient der Ermittlung der Zeitspanne zwischen Härterzugabe und Gelierung des Lackes als Maß für die Reaktivität des Systems.

Die Bestimmung der Trocknung erfolgte nach DIN EN ISO 9117-5:2012-11 auf Glas.

Die Lösungsmittelbeständigkeiten wurden nach DIN EN ISO 4628-1:2016-07 ermittelt. Für den Test der Lösemittelbeständigkeiten wurden die Lösemittel Xylol (folgend auch als "Xy" abgekürzt), Methoxypropylacetat (folgend auch als "MPA" abgekürzt), Ethylacetat (folgend auch als "EA" abgekürzt) und Aceton (folgend auch als "Ac" abgekürzt) verwendet. Die Kontaktzeit betrug jeweils 5 min. Die Abmusterung wurde entsprechend der angeführten Norm durchgeführt. Die Prüffläche wird visuell und durch Verkratzung beurteilt, dabei wird folgende Klassifikation vorgenommen: 0 = Keine Veränderung feststellbar; 1 = Quellungsring, Oberfläche hart, nur sichtbare Veränderung; 2 = Quellungsring, geringe Erweichung; 3 = deutliche Erweichung (evtl. geringe Blasenbildung); 4 = Starke Erweichung (evtl. starke Blasenbildung), durchritzbar bis zum Untergrund; 5 = Beschichtung komplett zerstört ohne Fremdeinwirkung.

Die Bestimmung der Pendeldämpfung nach König erfolgte nach DIN EN ISO 1522:2007-04 auf Glasplatten. Die Trockenfilmdicke betrug bei allen Filmen 45-52 μm.

Gitterschnittprüfung erfolgte nach DIN EN ISO 2409:2006-13.

Der Glanz bei 20° der erhaltenen Beschichtungen wurde reflektometrisch nach DIN EN ISO 2813:2015-02 bestimmt.

Verkratzung - Crockmeter:

Das Beschichtungsmaterial wird mittels Crockmeter entsprechend DIN EN ISO 105-X12:2016-11 verkratzt. Das beschichtete Substrat wird parallel zur Richtung des Reibzapfens gelegt. Mit einer Frequenz von einem Zyklus je Sekunde erfolgt 20-mal auf einer Länger von 104±3 mm auf der trockenen Probe eine geradlinige Reibbewegung mit 10 Hin- und 10 Herbewegungen und einer abwärtsgerichteten Kraft von 9±0,2 N. Anschließend wird der Glanz der Probe reflektometrisch bestimmt.

Reflow:

Das Rückfließverhalten (Reflow) bezeichnet die Erholung einer verkratzen Beschichtungsoberfläche nach Temperatureinwirkung bezogen auf den Glanzwert. Eine Beschichtung wird mittels Trockenverkratzung verkratzt (Crockmeter). Der Restglanz wird nach dem Verkratzungszyklus bestimmt. Die Beschichtung wird 2h, bei 60 °C in den Ofen gelegt und der Glanz anschließend nach dem zuvor beschriebenen Verfahren ermittelt. Der Reflow wird in Prozent angegeben, d.h. Verhältnis von Restglanz nach der thermischen Behandlung zu Glanz vor dem Verkratzen.

**Bewitterung:**

- CAM 180:
  Die Schnellbewitterungsstudien in Gegenwart von UV-Strahlung wurden entsprechend SAE J2527 durchgeführt. Die Prüfplatten wurden alle 250 h abgeprüft.

- UV-A-Test:
  Die UV-A Tests der Beschichtungsmaterialien wurden entsprechend DIN EN ISO 16474-3:2014-03 (Zyklus 1) durchgeführt. Die Prüfplatten wurden alle 250 h abgeprüft.
- b-Wert und Delta E Berechnung:
  Der Delta E Wert lässt sich aus den nach dem Farbenraum Lab nach DIN EN ISO/CIE 11664-4:2019-04 mittels "Dr. Lange - Micro Color II" bestimmten L, a und b Werten berechnen.

**Synthesen der erfindungsgemäßen Polyasparaginsäureester (PAS)**

PAS 1:

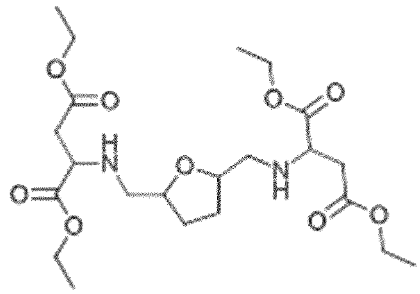

Hergestellt unter Verwendung von Tetrahydrofurandimethanamin

PAS 2:

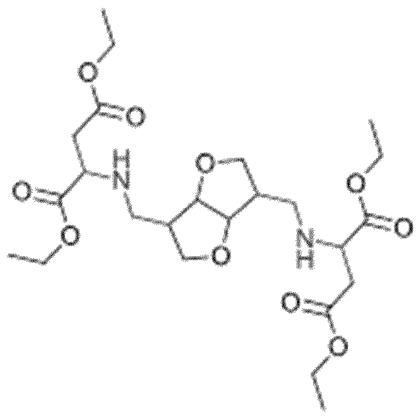

Hergestellt unter Verwendung von 3S,3aR,6S,6aR-6-(aminomethyl)-hexahydrofuro[3,2-b furan-3-yl]-methanamin.

PAS 3 und 4:

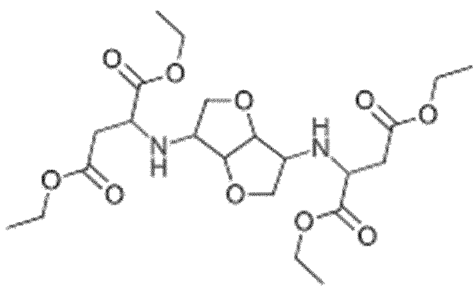

Hergestellt unter Verwendung von zwei Isomeren: PAS 3: 3S,3aR,6S,6aR-Hexahydrofuro[3,2-*b*]furan-3,6-diamin und PAS 4: 3R,3aR,6S,6aR-Hexahydrofuro[3,2-*b*]furan-3,6-diamin

**Polyasparaginsäureester PAS 1 (erfindungsgemäß)**

340.2g Tetrahydrofurandimethanamin (Rel-((2R,5S)-Tetrahydrofuran-2,5-diyl)dimethanamin, cis) wurden bei 30 °C unter trockenem Stickstoff und Rühren vorgelegt. Dazu wurden 900.0g Maleinsäurediethylester so zugetropft, dass die Temperatur nicht über 60 °C gestiegen ist. Nach der beendeten Zugabe wurde die Temperatur auf 45 °C eingestellt und der Ansatz wurde bei 45 °C 1 Stunde gerührt. Anschließend wurde der Ansatz bei 23°C 1 Woche gelagert. Man erhielt ein farbhelles Produkt mit folgenden Kenndaten:

| | |
|---|---|
| Fumarsäurediethylester (GC) | 1.74 Gew.-Prozent |
| Viskosität | 170 mPas |
| Farbzahl | 22 APHA |
| Aminzahl | 234 mg KOH/g |

**Polyasparaginsäureester PAS 3 (erfindungsgemäß)**

72.08g (3S,3aR,6S,6aR)-hexahydrofuro[3,2-b]furan-3,6-diamin wurden unter trockenem Stickstoff vorgelegt und unter Rühren auf 90°C erwärmt. Dazu wurden 172.0g Maleinsäurediethylester so zugetropft, dass die Temperatur nicht bei 90°C geblieben ist. Nach der beendeten Zugabe wurde die Temperatur auf 60 °C eingestellt und der Ansatz wurde bei 60 °C 2 Stunden gerührt. Anschließend wurde der Ansatz bei 23°C 20 Wochen gelagert. Man erhielt ein Produkt mit folgenden Kenndaten:

| | |
|---|---|
| Fumarsäurediethylester (GC) | 4.82 Gew.-Prozent |
| Aminzahl | 240 mg KOH/g |
| Viskosität | 1010 mPas |

**Polyasparaginsäureester PAS 2 (erfindungsgemäß)**

86.11g [(3S,3aR,6S,6aR)-6-(aminomethyl)-hexahydrofuro[3,2-b]furan-3-yl]methanamin wurden bei 30 °C unter trockenem Stickstoff und Rühren vorgelegt. Dazu wurden 172.0g Maleinsäurediethylester so zugetropft, dass die Temperatur nicht über 60 °C gestiegen ist. Nach der beendeten Zugabe wurde die Temperatur auf 45 °C eingestellt und der Ansatz wurde bei 45 °C eine Stunde gerührt. Anschließend wurde der Ansatz bei 23°C 4 Wochen gelagert. Man erhielt ein Produkt mit folgenden Kenndaten:

| | |
|---|---|
| Fumarsäurediethylester (GC) | 5.35 Gew.-Prozent |
| Aminzahl | 253 mg KOH/g |
| Viskosität | 850 mPas |

**Polyasparaginsäureester PAS 4 (erfindungsgemäß)**

72.08g 3R,3aR,6S,6aR-Hexahydrofuro[3,2-*b*]furan-3,6-diamin wurden unter trockenem Stickstoff vorgelegt und unter Rühren auf 90°C erwärmt. Dazu wurden 172.0g Maleinsäurediethylester so zugetropft, dass die Temperatur nicht über bei 90°C gestiegen ist. Nach der beendeten Zugabe wurde die Temperatur auf 60 °C eingestellt und der Ansatz wurde bei 60 °C 2 Stunden gerührt. Anschließend wurde der Ansatz bei 23°C 8 Wochen gelagert. Man erhielt ein Produkt mit folgenden Kenndaten:

| | |
|---|---|
| Fumarsäurediethylester (GC) | 8.75 Gew.-Prozent |
| Aminzahl | 226 mg KOH/g |
| Viskosität | 420 mPas |

**Herstellung der Beschichtungen:**

Tabelle 1 Einwaagen in Gramm

| | 1 (Vgl.) | 2 (Vgl.) | | 3 | 4 | 5 | 6 |
|---|---|---|---|---|---|---|---|
| **Stammlack:** | | | | | | | |
| PAS 1 | | | | 100,00 | | | |
| PAS 2 | | | | | 100,00 | | |
| PAS 3 | | | | | | 50,00 | |
| PAS 4 | | | | | | | 100 |
| Desmophen NH 1220 | 100,00 | 35,00 | | | | | |
| Desmophen NH 1420 | | 65,00 | | | | | |
| **Härter** | | | | | | | |
| Desmodur N 3900 | 76,50 | 68,93 | | 75,18 | 69,30 | 36,64 | 73,28 |
| gesamt | 176,50 | 168,93 | | 175,18 | 169,30 | 86,64 | 173,28 |
| | | | | | | | |
| Verhältnis (NCO/NH) | 1,0 | 1,0 | | 1,0 | 1,0 | 1,0 | 1,0 |
| Gelzeit in min | 1 min. | 11 min | | 5 min | 4 min | 162 min | 187 min |

**Vermischung des Stammlackes mit dem Härter und Applikation:**

Die oben aufgeführten Komponenten A (Stammlack) und B (Härter) wurden jeweils zusammengemischt und innig verrührt. Anschließend wurden die Mischungen mit einer Aufziehrahmen (Naßschichtdicke 90 $\mu$m) auf die Glasplatten appliziert und bei Raumtemperatur (23 °C) getrocknet. Es wurden brillante, hochglänzende Beschichtungen mit einer Trockenfilmschichtdicke von 45 bis 52 $\mu$m erhalten. Eine Übersicht über die ermittelten lacktechnischen Eigenschaften der Beschichtungen ist in Tabelle 2 bis 4 dargestellt.

Tabelle 2

| **Beispiel** | **1 (Vgl.)** | **2 (Vgl.)** | | **3** | **4** | **5** | **6** |
|---|---|---|---|---|---|---|---|
| **Trockenfilmschichtdicke ($\mu$m)** | 50 | 45 | | 45 | 52 | 50 | 52 |
| **Pendelhärte** | | | | | | | |
| nach 1 d | 52 | 174 | | 194 | 148 | 170 | 136 |
| nach 7 d | | | | 191 | 171 | 200 | 202 |
| **Lösemittelbeständigkeit nach 1 d** | | | | | | | |
| Xylol (1 / 5 min.) | 2/2 | 2/2 | | 1 / 1 | 1/2 | 1/1 | 2/3 |
| MPA (1 / 5 min.) | 2/3 | 2 / 2-3 | | 1/2 | 1 / 1 | 1/2 | 2/3 |
| Ethylacetat (1 / 5 min.) | 2-3 / 5 | 3/5 | | 2/3 | 2/3 | 2/4 | 4/4 |
| Aceton (1 / 5 min.) | 5/5 | 5/5 | | 3/4 | 4/4 | 3/4 | 4/5 |
| **Lösemittelbeständigkeit nach 7 d** | | | | | | | |
| Xylol (1 / 5 min.) | | | | 1/1 | 1 / 1 | 1/2 | 1/1 |
| MPA (1 / 5 min.) | | | | 1/2 | 1 / 1 | 2/2 | 1/1 |
| Ethylacetat (1 / 5 min.) | | | | 1/3 | 2/3 | 2/5 | 2/3 |
| Aceton (1 / 5 min.) | | | | 3/4 | 3/3 | 4/5 | 3/3 |
| Verkratzungsbeständigkeit -Crockmeter (Trockenverkratzung) | | | | | | | |
| Glanz 20° | Start | 86 | | 84 | 86 | n.b | n.b. |
| | nach Verkratzung | 5 | | 15 | 13 | n.b | n.b. |

(fortgesetzt)

| | | | | | | | |
|---|---|---|---|---|---|---|---|
| **Lösemittelbeständigkeit** | 2h 60°C Ofen | 21 | | 63 | 40 | n.b | n.b. |
| Rel. Glanzhaltung | | 24,4% | | 75% | 46,5% | n.b | n.b. |

**Lösemittelbeständigkeit:**

Gemessen an Klarlacken auf Glasplatte. Bewertung: 0 - 5 (0= Lackfilm unverändert; 5=völlig aufgelöst)

Wie aus der Tabelle ersichtlich ist, weisen Beschichtungen basieren auf den erfindungsgemäßen Polyasparaginsäureestern eine deutlich bessere Lösemittelbeständigkeit und eine deutlich besseres Reflow-Verhalten auf als Beschichtungen basierend auf Polyasparaginsäureestern des Standes der Technik.

**Bewitterungsversuche:**

i) Glanzentwicklung:

Tabelle3

| **UVA** | | | | | | | | | |
|---|---|---|---|---|---|---|---|---|---|
| h | 0 | 250 | 500 | 750 | 1000 | 1250 | 1500 | 1750 | \| 2000 |
| | Glanzentwicklung 20° | | | | | | | | |
| 1 | 88 | 93 | 93 | 91 | 89 | 89 | 82 | 87 | 87 |
| 3 | 89 | 76 | 93 | 90 | 88 | 88 | 88 | 90 | 89 |
| 2 (Vgl). | 88 | 93 | 94 | 92 | 90 | 88 | 86 | 88 | 90 |
| **CAM 180** | | | | | | | | | |
| h | 0 | 250 | \| 500 | 750 | 1000 | 1250 | 1500 | 1750 | \| 2000 |
| | Glanzentwicklung 20° | | | | | | | | |
| 1 | 87 | 88 | 88 | 82 | 87 | 89 | 83 | 86 | 86 |
| 3 | 85 | 90 | 91 | 88 | 89 | 87 | 89 | 86 | 0 |
| 2 (Vgl). | 85 | 76 | 68 | 78 | 87 | 0 | | | |

Wie aus Tabelle 3 ersichtlich ist, ist die Glanzentwicklung der erfindungsgemäßen Beschichtungen vergleichbar zu Beschichtungen basierend auf Polyasparaginsäureestern des Standes der Technik.

ii) Vergilbungsbeständigkeit:

Tabelle 4

| **UVA**: | | | | | | | | | | | | | | | | | | |
|---|---|---|---|---|---|---|---|---|---|---|---|---|---|---|---|---|---|---|
| h | 0 | 250 | 500 | 750 | 1000 | 1250 | 1500 | 1750 | 2000 | 0 | 250 | 500 | 750 | 1000 | 1250 | 1500 | 1750 | 2000 |
| | **b-Wert** | | | | | | | | | **Delta E** | | | | | | | | |
| 1 | -0.9 | 0.6 | 1.3 | 3.2 | 4.7 | 5.5 | 5.6 | 5.7 | 6 | 0 | 1,8 | 2,5 | 4,4 | 6 | 6,7 | 6,9 | 7 | 7,4 |
| 3 | 8.9 | 13.6 | 13 | 13.5 | 12.6 | 12.6 | 12.6 | 12.2 | 11.3 | 0 | 5,4 | 5 | 5,4 | 5,6 | 6,1 | 6,2 | 5,8 | 5,5 |
| 2 | -0.4 | 2.1 | 1.9 | 3.3 | 5.2 | 6.8 | 7.8 | 8.4 | 9 | 0 | 2,6 | 2,5 | 3,9 | 5,8 | 7,5 | 8,5 | 9,1 | 9,8 |
| **CAM 180:** | | | | | | | | | | | | | | | | | | |
| h | 0 | 250 | 500 | 750 | 1000 | 1250 | 1500 | 1750 | 2000 | 0 | 250 | 500 | 750 | 1000 | 1250 | 1500 | 1750 | 2000 |
| | **b-Wert** | | | | | | | | | **Delta E** | | | | | | | | |
| 1 | -0.4 | 1.2 | 2.6 | 3 | 3 | 2.9 | 2.8 | 2.8 | 2.9 | 0 | 1,7 | 3,2 | 3,6 | 3,6 | 3,5 | 3,4 | 3,4 | 3,5 |
| 3 | 4.9 | 3.3 | 3.2 | 3.1 | 3.3 | 3.8 | 3.4 | 3.2 | 3.2 | 0 | 0,6 | 0,4 | 1,2 | 0,7 | 0,8 | 0,8 | 0,9 | |
| 2 | -0.3 | 0.9 | 1.3 | 2.7 | 2.7 | 3.4 | 2.1 | 2.6 | 4.6 | 0 | 1,3 | 1,8 | 3,2 | 3,4 | 4,4 | 2,6 | 3,2 | 5,5 |

Wie aus Tabelle 4 ersichtlich, weisen die erfindungsgemäßen Beschichtungen eine deutlich bessere Vergilbungsbeständigkeit auf als entsprechende Polyasparat-basierte Beschichtungen des Standes der Technik.

## Patentansprüche

1. Zusammensetzungen A1 enthaltend oder bestehend aus einen/einem oder mehrere/n Polyasparaginsäureester/n der allgemeinen Formel (I)

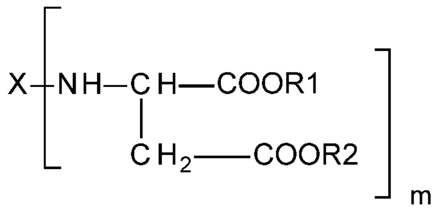

(I),

in welcher

X für einen m-wertigen organischen Rest steht, der erhalten werden kann durch Entfernung der primären Aminogruppen aus cyclischen Ethern, bei denen es sich um Monocyclen oder um anellierte Bicyclen auf Basis von monocyclischen Ethern handelt und die an wenigstens 2 der Ringkohlenstoffatome eine Gruppe tragen, die ausgewählt ist aus primärer Aminogruppe und aliphatisch gebundener primärer Aminogruppe, wobei
R1 und R2 für gleiche oder verschiedene organische Reste, mit je 1 bis 18 Kohlenstoffatomen stehen, und
m für eine ganze Zahl > 1 steht,

und
gegebenenfalls einen/einem oder mehrere/n Polyasparaginsäureester/n mit primärer Aminogruppe der allgemeinen Formel (II)

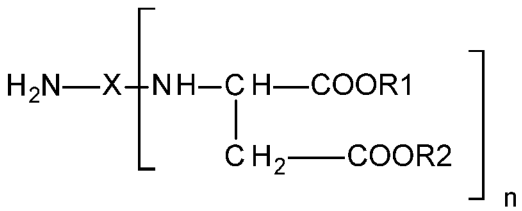

(II)

in welcher

n für m-1 steht und
X und die Reste R1 und R2 die oben genannten Bedeutungen haben.

2. Verfahren zur Herstellung von Zusammensetzungen A1 enthaltend oder bestehend aus einen/einem oder mehrere/n Polyasparaginsäureester/n der allgemeinen Formel (I)

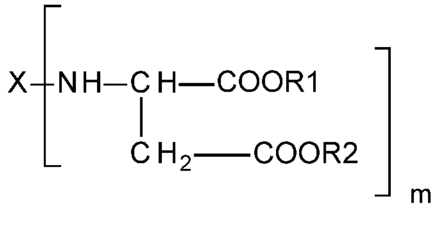

(I),

in welcher

X für einen m-wertigen organischen Rest steht, der erhalten werden kann durch Entfernung der primären Aminogruppen aus cyclischen Ethern, bei denen es sich um Monocyclen oder um anellierte Bicyclen auf Basis von monocyclischen Ethern handelt und die an wenigstens 2 der Ringkohlenstoffatome eine Gruppe tragen, die ausgewählt ist aus primärer Aminogruppe und aliphatisch gebundener primärer Aminogruppe, wobei
R1 und R2 für gleiche oder verschiedene organische Reste, mit je 1 bis 18 Kohlenstoffatomen stehen, und
m für eine ganze Zahl > 1 steht,

und
gegebenenfalls einen/einem oder mehrere/n Polyasparaginsäureester/n mit primärer Aminogruppe der allgemeinen Formel (II)

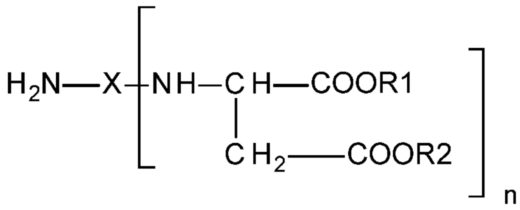

(II)

in welcher

n für m-1 steht und
X und die Reste R1 und R2 die oben genannten Bedeutungen haben,

durch Umsetzung von Polyaminen der allgemeinen Formel (V)

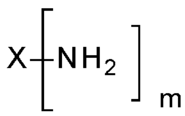

(V),

wobei X und m die oben genannte Bedeutung haben
mit Verbindungen der allgemeinen Formel (VI)

R1 OOC-C H=C H-COOR2 (VI),

wobei R1 und R2 die oben genannte Bedeutung haben.

**3.** Zusammensetzungen A1 gemäß Anspruch 1 oder Verfahren zur Herstellung von Zusammensetzungen A1 gemäß Anspruch 2, wobei es sich bei den cyclischen Ethern, von denen sich X ableitet und die an wenigstens 2 der Ringkohlenstoffatome eine Gruppe tragen, die ausgewählt ist aus primärer Aminogruppe und aliphatisch gebundener primärer Aminogruppe, d.h. wobei es sich bei den Verbindungen der Formel V, um Monocyclen oder Bicyclen gemäß der folgenden allgemeinen Formeln III und IV handelt:

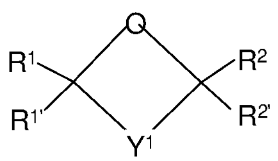

Formel III,

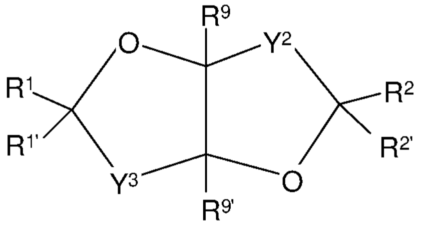

Formel IV,

wobei $Y^1$ für

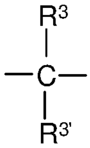 $Y^1a$ , 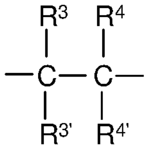 $Y^1b$ , 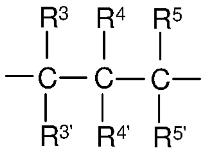 $Y^1c$ ,

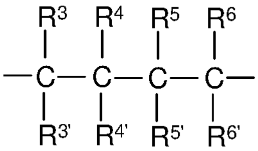 $Y^1d$ , 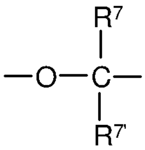 $Y^1e$ , 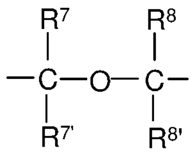 $Y^1f$ ,

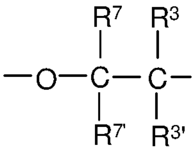 $Y^1g$ , 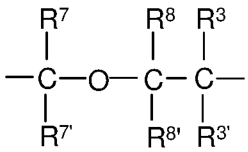 $Y^1h$ , 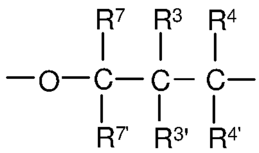 $Y^1i$

und $Y^2$ für

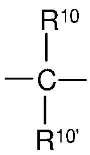 $Y^2a$ , 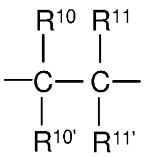 $Y^2b$ , 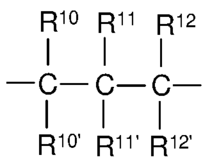 $Y^2c$ ,

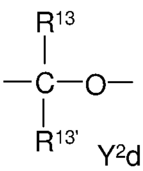 $Y^2d$ , 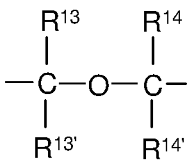 $Y^2e$ , 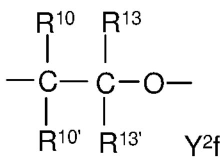 $Y^2f$

und $Y^3$ für

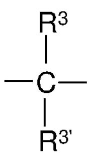 $Y^3a$ , 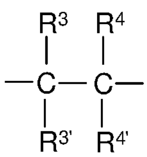 $Y^3b$ , 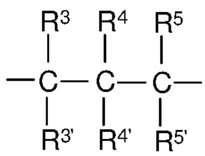 $Y^3c$ ,

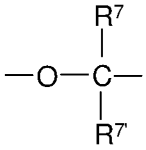 $Y^3d$ , 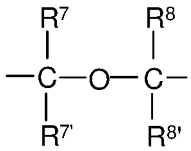 $Y^3e$ , 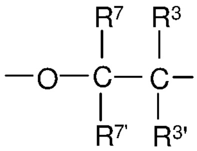 $Y^3f$

stehen, und

$R^1$, $R^2$, $R^7$, $R^8$, $R^{13}$, $R^{14}$ unabhängig voneinander für eine Alkylengruppe mit 1 bis 6 Kohlenstoffatomen oder Wasserstoff oder organische Reste stehen, wobei es sich bei letzteren um gesättigte oder ungesättigte, lineare oder verzweigte, aliphatische oder cycloaliphatische oder gegebenenfalls substituierte aromatische oder araliphatische einbindige Reste mit bis zu 18 Kohlenstoffatomen, die gegebenenfalls Heteroatome aus der Reihe Sauerstoff, Schwefel, Stickstoff enthalten können, handelt, wobei zu den Heteroatom-enthaltenden Resten z.B. solche gehören, die gegenüber Isocyanatgruppen reaktive und/oder bei Temperaturen bis 100°C inerte funktionelle Gruppen enthalten (ausgenommen primäre Aminogruppen),
und
$R^3$, $R^4$, $R^5$, $R^6$, $R^{10}$, $R^{11}$, $R^{12}$ unabhängig voneinander für eine lineare oder verzweigte Alkylengruppe mit 1 bis 6 Kohlenstoffatomen oder eine Bindung oder Wasserstoff oder organische Reste stehen, wobei es sich bei letzteren um gesättigte oder ungesättigte, lineare oder verzweigte, aliphatische oder cycloaliphatische oder gegebenenfalls substituierte aromatische oder araliphatische einbindige Reste mit bis zu 18 Kohlenstoffatomen, die gegebenenfalls Heteroatome aus der Reihe Sauerstoff, Schwefel, Stickstoff enthalten können, handelt, wobei zu den Heteroatom-enthaltenden Resten z.B. solche gehören, die gegenüber Isocyanatgruppen reaktive und/oder bei Temperaturen bis 100°C inerte funktionelle Gruppen enthalten (ausgenommen primäre Aminogruppen),
wobei
im Fall der Formel III mindestens 2 der Reste $R^1$ bis $R^8$ an eine $NH_2$-Gruppe gebunden sind, und
im Fall der Formel IV mindestens einer der Reste $R^1$ bis $R^8$ an eine $NH_2$-Gruppe gebunden ist und mindestens einer der Reste $R^{10}$ bis $R^{14}$ an eine $NH_2$-Gruppe gebunden ist,
und
$R^9$ und $R^{9'}$ unabhängig voneinander für H oder einen Methylrest stehen,
und
$R^{1'}$ bis $R^{8'}$ und $R^{10'}$ bis $R^{14'}$ unabhängig voneinander für Wasserstoff oder organische Reste stehen, wobei es sich bei letzteren um gesättigte oder ungesättigte, lineare oder verzweigte, aliphatische oder cycloaliphatische oder gegebenenfalls substituierte aromatische oder araliphatische einbindige Reste mit bis zu 18 Kohlenstoffatomen, die gegebenenfalls Heteroatome aus der Reihe Sauerstoff, Schwefel, Stickstoff enthalten können, handelt, wobei zu den Heteroatom-enthaltenden Resten z.B. solche gehören, die gegenüber Isocyanatgruppen reaktive und/oder bei Temperaturen bis 100°C inerte funktionelle Gruppen enthalten (ausgenommen primäre Aminogruppen).

**4.** Zusammensetzungen A1 gemäß Anspruch 3 oder Verfahren zur Herstellung von Zusammensetzungen A1 gemäß Anspruch 3, wobei es sich bei den Alkylengruppen mit 1 bis 6 Kohlenstoffatomen um $CH_2$, $CH_2$-$CH_2$, $CH_2$-$CH_2$-$CH_2$ oder $CH_2$-$CH_2$-$CH_2$-$CH_2$, bevorzugt um $CH_2$ handelt.

**5.** Zusammensetzungen A1 gemäß Anspruch 3 oder 4 oder Verfahren zur Herstellung von Zusammensetzungen A1 gemäß Anspruch 3 oder 4, wobei die Reste $R^1$, $R^2$, $R^3$, $R^4$, $R^5$, $R^6$, $R^7$, $R^8$, $R^{10}$, $R^{11}$, $R^{12}$ $R^{13}$, $R^{14}$ -falls sie für Wasserstoff oder organische Reste stehen- unabhängig voneinander für Wasserstoff und/oder Alkylreste mit je 1 bis 8 Kohlenstoffatomen stehen.

**6.** Zusammensetzungen A1 gemäß einem der Ansprüche 1 oder 3 bis 5 oder Verfahren zur Herstellung von Zusammensetzungen A1 gemäß einem der Ansprüche 2 bis 5, bei denen X erhalten wurde durch Entfernung der primären Aminogruppen aus cyclischen Ethern der oben beschriebenen Art, die auf Ausgangstoffen basieren, die biobasiert erhalten wurden.

**7.** Zusammensetzungen A1 gemäß einem der Ansprüche 1 oder 3 bis 6 in Mischung mit weiteren, von A1 verschiedenen Polyasparaginsäureestern oder Polyasparaginsäureester-enthaltenden bzw. daraus bestehenden Zusammensetzungen (Komponente A2).

**8.** Verwendung von Zusammensetzungen A1 gemäß einem der Ansprüche 1 oder 3 bis 7 zur Herstellung von Beschichtungszuammensetzungen, bevorzugt zweikomponentigen Beschichtungszusammensetzungen (2K-Beschichtungsmittel).

**9.** Beschichtungszusammensetzungen, bevorzugt zweikomponentige Beschichtungszusammensetzungen (2K-Beschichtungsmittel), enthaltend

a1) wenigstens eine Polyasparaginsäureester-haltige Zusammensetzung A1 gemäß einem der Ansprüche 1 oder 3 bis 6,
a2) gegebenenfalls weitere, von A1 verschiedene Polyasparaginsäureester oder Polyasparaginsäureester-enthaltende bzw. daraus bestehende Zusammensetzungen A2,
b) mindestens eine Polyisocyanatkomponente B
c) gegebenenfalls eine oder mehrere, von A1 und A2 verschiedene, gegenüber Isocyanatgruppen reaktionsfähige Komponenten C,
d) gegebenenfalls Hilfs- und Zusatzstoffe (Komponente D).

**10.** Verfahren zum Beschichten eines Substrats, welches mindestens folgende Schritte umfasst:

i) Aufbringen einer zweikomponentigen Beschichtungszusammensetzung gemäß Anspruch 9 auf mindestens einen Teil eines zu beschichtenden Substrats und
ii) Härten der Beschichtungszusammensetzung aus Schritt i).

**11.** Substrate beschichtet mit einer Beschichtung, erhältlich nach einem Verfahren gemäß Anspruch 10.

**Claims**

**1.** Compositions A1 comprising or consisting of one or more polyaspartic esters of the general formula (I)

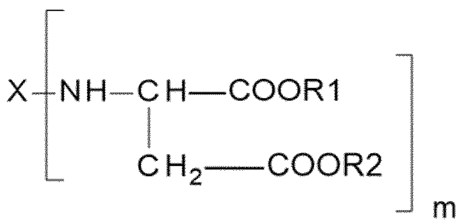

(I),

in which

X is an m-valent organic radical that can be obtained by removing the primary amino groups from cyclic ethers, said ethers being monocycles or fused bicycles based on monocyclic ethers and which on at least 2 of the ring carbon atoms bear a group selected from primary amino group and aliphatically attached primary amino group, where
R1 and R2 are identical or different organic radicals each having 1 to 18 carbon atoms and
m is an integer > 1,

and
optionally one or more polyaspartic esters having a primary amino group of the general formula (II)

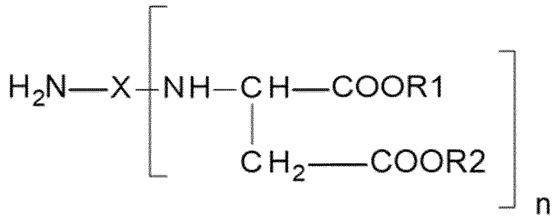

(II)

in which

n is m-1 and
X and the radicals R1 and R2 are as defined above.

**2.** Process for producing compositions A1 comprising or consisting of one or more polyaspartic esters of the general formula (I)

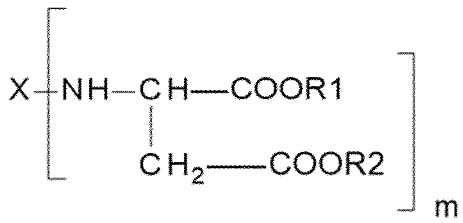

(I),

in which

X is an m-valent organic radical that can be obtained by removing the primary amino groups from cyclic ethers, said ethers being monocycles or fused bicycles based on monocyclic ethers and which on at least 2 of the ring carbon atoms bear a group selected from primary amino group and aliphatically attached primary amino group, where
R1 and R2 are identical or different organic radicals each having 1 to 18 carbon atoms and
m is an integer > 1,

and
optionally one or more polyaspartic esters having a primary amino group of the general formula (II)

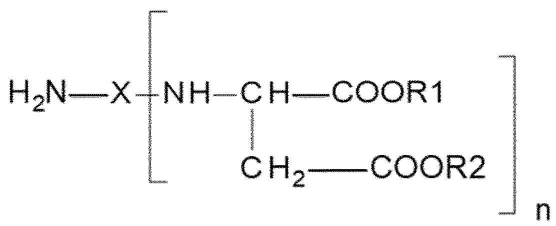

(II)

in which

n is m-1 and
X and the radicals R1 and R2 are as defined above,

by reacting polyamines of the general formula (V)

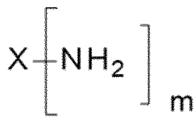

(V),

in which X and m are as defined above,
with compounds of the general formula (VI)

R1OOC-C H=C H-COOR2 (VI),

where R1 and R2 are as defined above.

3. Compositions A1 according to Claim 1 or process for producing compositions A1 according to Claim 2, wherein the cyclic ethers from which X is derived and which on at least 2 of the ring carbon atoms bear a group selected from primary amino group and aliphatically attached primary amino group, i.e. the compounds of the formula V, are monocycles or bicycles according to the following general formulas III and IV:

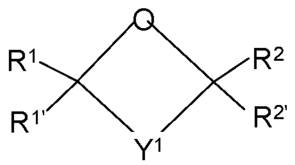

Formula III,

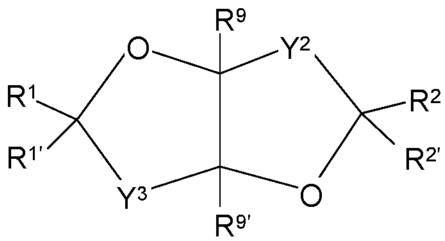

Formula IV,

where $Y^1$ is

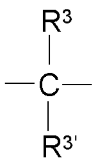
$Y^1a$ ,
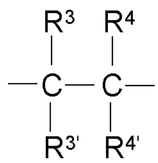
$Y^1b$ ,
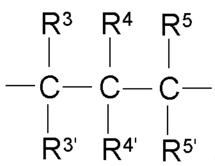
$Y^1c$ ,

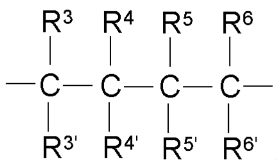
$Y^1d$ ,
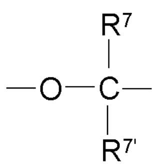
$Y^1e$ ,
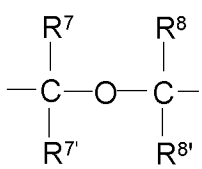
$Y^1f$ ,

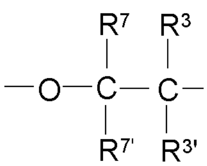
$Y^1g$ ,
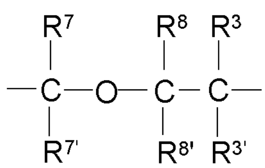
$Y^1h$ ,
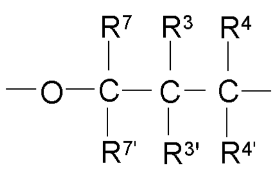
$Y^1i$

and $Y^2$ is

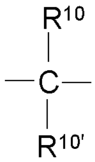 Y2a , 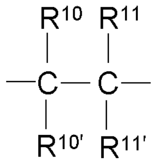 Y2b , 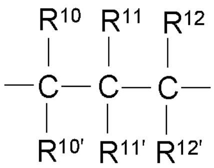 Y2c ,

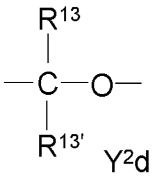 Y2d , 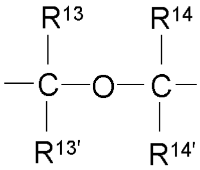 Y2e , 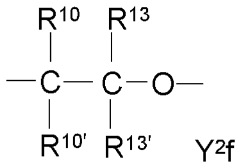 Y2f

and $Y^3$ is

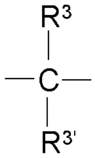 Y3a , 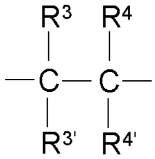 Y3b , 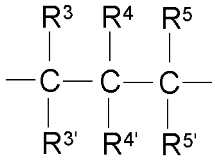 Y3c ,

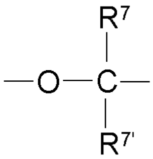 Y3d , 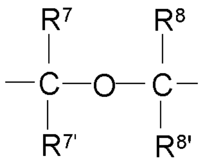 Y3e , 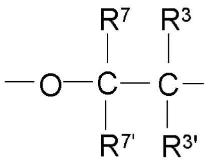 Y3f

and

$R^1$, $R^2$, $R^7$, $R^8$, $R^{13}$, $R^{14}$ are independently an alkylene group having 1 to 6 carbon atoms or hydrogen or organic radicals, the latter being saturated or unsaturated, linear or branched, aliphatic or cycloaliphatic or optionally substituted aromatic or araliphatic monovalent radicals having up to 18 carbon atoms, which may optionally contain heteroatoms from the series oxygen, sulfur and nitrogen, the heteroatom-containing radicals including e.g. those that contain functional groups reactive toward isocyanate groups and/or functional groups inert toward isocyanate groups at temperatures up to 100°C (primary amino groups excepted),
and
$R^3$, $R^4$, $R^5$, $R^6$, $R^{10}$, $R^{11}$, $R^{12}$ are independently a linear or branched alkylene group having 1 to 6 carbon atoms or a bond or hydrogen or organic radicals, the latter being saturated or unsaturated, linear or branched, aliphatic or cycloaliphatic or optionally substituted aromatic or araliphatic monovalent radicals having up to 18 carbon atoms, which may optionally contain heteroatoms from the series oxygen, sulfur and nitrogen, the heteroatom-containing radicals including e.g. those that contain functional groups reactive toward isocyanate groups and/or functional groups inert toward isocyanate groups at temperatures up to 100°C (primary amino groups excepted), where
in the case of formula III at least 2 of the radicals $R^1$ to $R^8$ are attached to a $NH_2$ group, and
in the case of formula IV at least one of the radicals $R^1$ to $R^8$ is attached to a $NH_2$ group and at least one of the radicals $R^{10}$ to $R^{14}$ is attached to a $NH_2$ group, and
$R^9$ and $R^{9'}$ are independently H or a methyl radical,
and
$R^{1'}$ to $R^{8'}$ and $R^{10'}$ to $R^{14'}$ are independently hydrogen or organic radicals, the latter being saturated or unsaturated, linear or branched, aliphatic or cycloaliphatic or optionally substituted aromatic or araliphatic monovalent radicals having up to 18 carbon atoms, which may optionally contain heteroatoms from the series oxygen, sulfur and nitrogen, the heteroatom-containing radicals including e.g. those that contain functional groups reactive toward isocyanate groups and/or functional groups inert toward isocyanate groups at temperatures up to 100°C

(primary amino groups excepted).

4. Compositions A1 according to Claim 3 or process for producing compositions A1 according to Claim 3, wherein the alkylene groups having 1 to 6 carbon atoms are $CH_2$, $CH_2$-$CH_2$, $CH_2$-$CH_2$-$CH_2$ or $CH_2$-$CH_2$-$CH_2$-$CH_2$, preferably $CH_2$.

5. Compositions A1 according to Claim 3 or 4 or process for producing compositions A1 according to Claim 3 or 4, wherein the radicals $R^1$, $R^2$, $R^3$, $R^4$, $R^5$, $R^6$, $R^7$, $R^8$, $R^{10}$, $R^{11}$, $R^{12}$, $R^{13}$, $R^{14}$, where they are hydrogen or organic radicals, are independently hydrogen and/or alkyl radicals each having 1 to 8 carbon atoms.

6. Compositions A1 according to any of Claims 1 or 3 to 5 or process for producing compositions A1 according to any of Claims 2 to 5, in which X was obtained by removing the primary amino groups from cyclic ethers of the type described above that are based on starting materials obtained in a biobased manner.

7. Compositions A1 according to any of Claims 1 or 3 to 6 in a mixture with further polyaspartic esters different from A1 or compositions comprising or consisting of polyaspartic esters (component A2).

8. Use of compositions A1 according to any of Claims 1 or 3 to 7 in the production of coating compositions, preferably two-component coating compositions (2C coating compositions).

9. Coating compositions, preferably two-component coating compositions (2C coating compositions), comprising

   a1) at least one polyaspartic ester-containing composition A1 according to any of claims 1 or 3 to 6,
   a2) optionally further polyaspartic esters different from A1 or compositions A2 comprising or consisting of polyaspartic esters,
   b) at least one polyisocyanate component B,
   c) optionally one or more components C different from A1 and A2 and reactive toward isocyanate groups,
   d) optionally auxiliaries and additives (component D).

10. Process for coating a substrate that comprises at least the following steps:

   i) applying a two-component coating composition according to Claim 9 to at least part of a substrate to be coated and
   ii) curing the coating composition from step i).

11. Substrates coated with a coating obtainable in accordance with a process according to Claim 10.

**Revendications**

1. Compositions A1 contenant ou constituées (d')un ou plusieurs poly(esters d'acide aspartique) de formule générale (I)

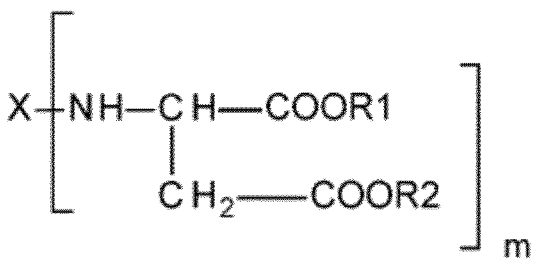

(I),

dans laquelle

X représente un radical organique m valent qui peut être obtenu par élimination des groupes amino primaire d'éthers cycliques, qui sont des monocycles ou des bicycles condensés à base d'éthers monocycliques et qui portent un groupe au niveau d'au moins 2 des atomes de carbone de cycle, qui est choisi parmi un groupe amino primaire et un groupe amino primaire lié de manière aliphatique,

R1 et R2 représentent des radicaux organiques identiques ou différents, comportant chacun 1 à 18 atomes de carbone, et
m représente un nombre entier > 1,

et éventuellement (d')un ou plusieurs poly(esters d'acide aspartique) comportant un groupe amino primaire de formule générale (II)

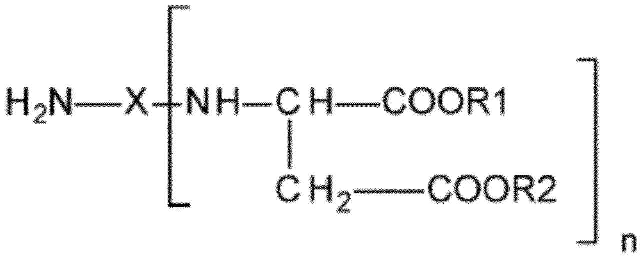

(II)

dans laquelle

n représente m - 1 et
X et les radicaux R1 et R2 possèdent les significations mentionnées ci-dessus.

**2.** Procédé de préparation de compositions A1 contenant ou constituées (d')un ou plusieurs poly(esters d'acide aspartique) de formule générale (I)

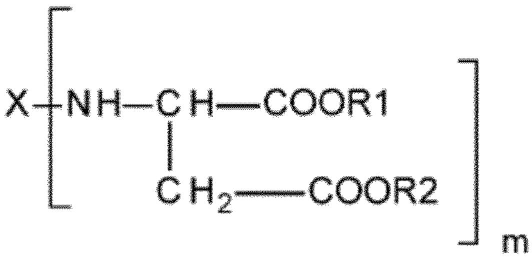

(I),

dans laquelle

X représente un radical organique m valent qui peut être obtenu par élimination des groupes amino primaire d'éthers cycliques, qui sont des monocycles ou des bicycles condensés à base d'éthers monocycliques et qui portent un groupe au niveau d'au moins 2 des atomes de carbone de cycle, qui est choisi parmi un groupe amino primaire et un groupe amino primaire lié de manière aliphatique,
R1 et R2 représentent des radicaux organiques identiques ou différents, comportant chacun 1 à 18 atomes de carbone, et
m représente un nombre entier > 1,

et
éventuellement (d')un ou plusieurs poly(esters d'acide aspartique) comportant un groupe amino primaire de formule générale (II)

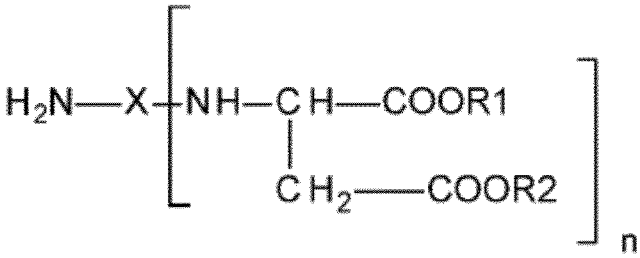

(II)

dans laquelle

n représente m - 1 et
X et les radicaux R1 et R2 possèdent les significations mentionnées ci-dessus,
par transformation de polyamines de formule générale (V)

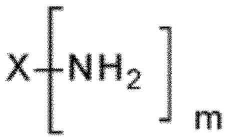

(V),

X et m possédant la signification mentionnée ci-dessus avec des composés de formule générale (VI)

R1OOC-CH=CH-COOR2 (VI),

R1 et R2 possédant la signification mentionnée ci-dessus.

**3.** Compositions A1 selon la revendication 1 ou procédé de préparation de compositions A1 selon la revendication 2, les éthers cycliques, desquels X est issu et qui portent un groupe au niveau d'au moins 2 des atomes de carbone de cycle, qui est choisi parmi un groupe amino primaire et un groupe amino primaire lié de manière aliphatique, c'est-à-dire les composés de formule V étant des monocycles ou des bicycles selon les formules générales suivantes III et IV :

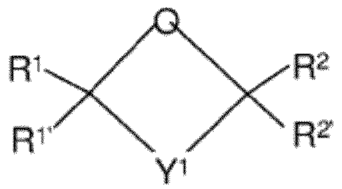

formule III,

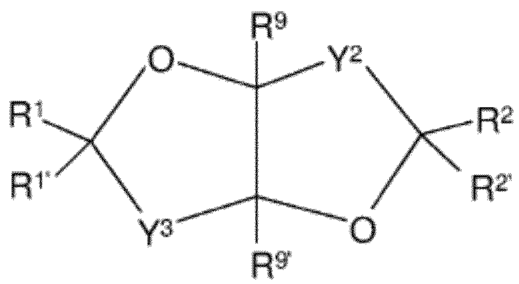

formule IV,

$Y^1$ représentant

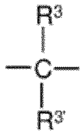 Y¹a , 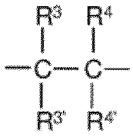 Y¹b , 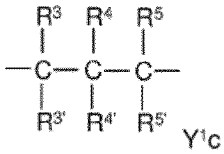 Y¹c ,

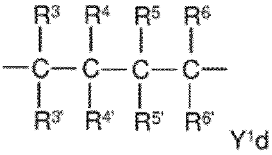 Y¹d , 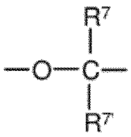 Y¹e , 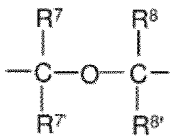 Y¹f ,

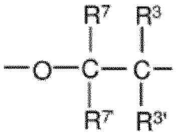 Y¹g , 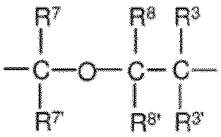 Y¹h , 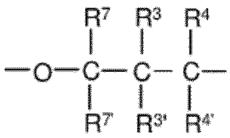 Y¹i

et $Y^2$ représentant

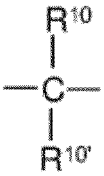 Y²a , 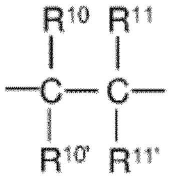 Y²b , 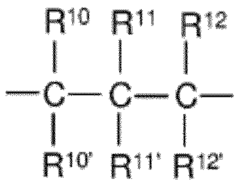 Y²c ,

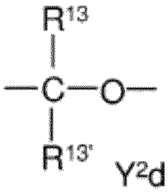 Y²d , 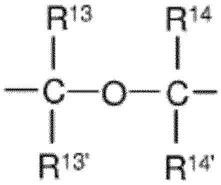 Y²e , 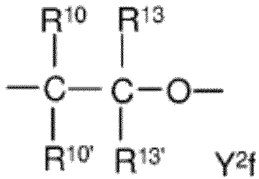 Y²f

et $Y^3$ représentant

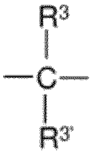 Y³a , 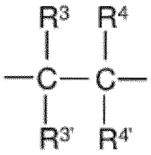 Y³b , 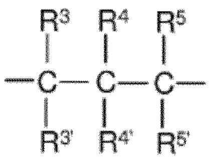 Y³c ,

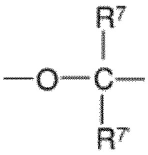 Y³d , 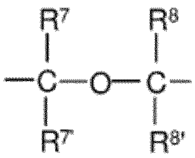 Y³e , 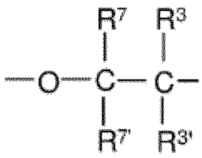 Y³f ,

et

$R^1$, $R^2$, $R^7$, $R^8$, $R^{13}$, $R^{14}$ représentant indépendamment les uns des autres, un groupe alkylène comportant 1 à 6 atomes de carbone ou hydrogène ou des radicaux organiques, ces derniers étant des radicaux monovalents

saturés ou insaturés, linéaires ou ramifiés, aromatiques ou araliphatiques éventuellement substitués ou aliphatiques ou cycloaliphatiques, comportant jusqu'à 18 atomes de carbone, qui peuvent éventuellement contenir des hétéroatomes de la série de l'oxygène, du soufre et de l'azote, les radicaux contenant des hétéroatomes comprenant par ex. ceux qui contiennent des groupes fonctionnels réactifs vis-à-vis des groupes isocyanate et/ou des groupes fonctionnels inertes vis-à-vis des groupes isocyanate à des températures allant jusqu'à 100°C (à l'exception des groupes amino primaires),

$R^3$, $R^4$, $R^5$, $R^6$, $R^{10}$, $R^{11}$, $R^{12}$ représentant, indépendamment les uns des autres, un groupe alkylène linéaire ou ramifié comportant de 1 à 6 atomes de carbone ou une liaison ou hydrogène ou des radicaux organiques, ces derniers étant des radicaux monovalents saturés ou insaturés, linéaires ou ramifiés, aromatiques ou araliphatiques éventuellement substitués ou aliphatiques ou cycloaliphatiques, comportant jusqu'à 18 atomes de carbone, qui peuvent éventuellement contenir des hétéroatomes de la série de l'oxygène, du soufre et de l'azote, les radicaux contenant des hétéroatomes comprenant par ex. ceux qui contiennent des groupes fonctionnels réactifs vis-à-vis des groupes isocyanate et/ou des groupes fonctionnels inertes vis-à-vis des groupes isocyanate à des températures allant jusqu'à 100°C (à l'exception des groupes amino primaires),

dans le cas de la formule III, au moins 2 des radicaux $R^1$ à $R^8$ étant liés à un groupe $NH_2$, et dans le cas de la formule IV, au moins un des radicaux $R^1$ à $R^8$ étant lié à un groupe $NH_2$ et au moins un des radicaux $R^{10}$ à $R^{14}$ étant lié à un groupe $NH_2$,

et

$R^9$ et $R^{9'}$ représentant indépendamment l'un de l'autre H ou un radical méthyle,

et

$R^{1'}$ à $R^{8'}$, et $R^{10'}$ à $R^{14'}$ représentant indépendamment les uns des autres hydrogène ou des radicaux organiques, ces derniers étant des radicaux monovalents saturés ou insaturés, linéaires ou ramifiés, aromatiques ou araliphatiques éventuellement substitués ou aliphatiques ou cycloaliphatiques, comportant jusqu'à 18 atomes de carbone, qui peuvent éventuellement contenir des hétéroatomes de la série de l'oxygène, du soufre et de l'azote, les radicaux contenant des hétéroatomes comprenant par ex. ceux qui contiennent des groupes fonctionnels réactifs vis-à-vis des groupes isocyanate et/ou des groupes fonctionnels inertes vis-à-vis des groupes isocyanate à des températures allant jusqu'à 100°C (à l'exception des groupes amino primaires).

**4.** Compositions A1 selon la revendication 3 ou procédé de préparation de compositions A1 selon la revendication 3, les groupes alkylène comportant 1 à 6 atomes de carbone étant $CH_2$, $CH_2$-$CH_2$, $CH_2$-$CH_2$-$CH_2$ ou $CH_2$-$CH_2$-$CH_2$-$CH_2$, préférablement $CH_2$ .

**5.** Compositions A1 selon la revendication 3 ou 4 ou procédé de préparation de compositions A1 selon la revendication 3 ou 4, les radicaux $R^1$, $R^2$, $R^3$, $R^4$, $R^5$, $R^6$, $R^7$, $R^8$, $R^{10}$, $R^{11}$, $R^{12}$, $R^{13}$, $R^{14}$ - s'ils représentent hydrogène ou des radicaux organiques - représentant indépendamment les uns des autres hydrogène et/ou des radicaux alkyle comportant à chaque fois 1 à 8 atomes de carbone.

**6.** Compositions A1 selon l'une quelconque des revendications 1 ou 3 à 5 ou procédé de préparation de compositions A1 selon l'une quelconque des revendications 2 à 5, où X a été obtenu par élimination des groupes amino primaire d'éthers cycliques de la sorte décrite ci-dessus, qui sont à base de matières premières qui ont été obtenues de manière biosourcées.

**7.** Compositions A1 selon l'une quelconque des revendications 1 ou 3 à 6 en mélange avec d'autres poly(esters d'acide aspartique) ou compositions contenant ou constituées de poly(esters d'acide aspartique) (composant A2), différent(e)s de A1.

**8.** Utilisation de compositions A1 selon l'une quelconque des revendications 1 ou 3 à 7 pour la préparation de compositions de revêtement, préférablement de compositions de revêtement à deux composants (agents de revêtement 2K).

**9.** Compositions de revêtement, préférablement compositions de revêtement à deux composants (agents de revêtement 2K), contenant

a1) au moins une composition A1 contenant des poly(esters d'acide aspartique) selon l'une quelconque des revendications 1 ou 3 à 6,

a2) éventuellement d'autres poly(esters d'acide aspartique) ou compositions contenant ou constituées de poly(esters d'acide aspartique) A2, différent(e)s de A1,

b) au moins un composant polyisocyanate B

c) éventuellement un ou plusieurs composants C réactifs envers des groupes isocyanate, différents de A1 et A2,
d) éventuellement des auxiliaires et des additifs (composant D).

**10.** Procédé de revêtement d'un substrat qui comprend au moins les étapes suivantes:

i) application d'une composition de revêtement à deux composants selon la revendication 9 sur au moins une partie d'un substrat à revêtir et
ii) durcissement de la composition de revêtement de l'étape i) .

**11.** Substrats revêtus par un revêtement, obtenu par un procédé selon la revendication 10.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 0403921 A
- EP 0639628 A
- EP 0667362 A
- EP 0689881 A
- US 5214086 A
- EP 0699696 A
- EP 0596360 A
- EP 0893458 A
- DE 19701835
- EP 0470461 A
- WO 15130501 A
- WO 15130502 A
- US 5243012 A
- EP 141062 A
- EP 19158880
- EP 19158883


### In der Beschreibung aufgeführte Nicht-Patentliteratur


- **HOUBEN-WEYL.** *Meth. d. Org. Chemie,* 1957, 272
- *Usp. Khim.,* 1969, vol. 38, 1933
- *ChemSusChem,* 2011, vol. 4, 1823-1829